# EUROPEAN PATENT APPLICATION

(11) **EP 4 276 096 A1**
(43) Date of publication of application: **15.11.2023**
(21) Application number: 22736778.6
(22) Date of filing: 07.01.2022
(51) Int. Cl.: C07D 311/58, C07D 307/79

(54) **NOVEL GUANIDINE DERIVATIVE AND METHOD FOR PRODUCING SAME**

(30) Priority: 07.01.2021 JP 2021001758
(71) Applicant: PeptiDream Inc., Kawasaki-shi, Kanagawa 210-0821 (JP)
(72) Inventor: EHARA Takeru, Kawasaki-shi, Kanagawa 210-0821 (JP); TAKUWA Masatoshi, Kawasaki-shi, Kanagawa 210-0821 (JP); KURASAKI Haruaki, Kawasaki-shi, Kanagawa 210-0821 (JP); MORIMOTO Kouki, Kawasaki-shi, Kanagawa 210-0821 (JP); MATSUI Katsuma, Kawasaki-shi, Kanagawa 210-0821 (JP); MATSUDA Ayumu, Kawasaki-shi, Kanagawa 210-0821 (JP); OKADA Naoki, Kawasaki-shi, Kanagawa 210-0821 (JP); MATSUMOTO Masatoshi, Kawasaki-shi, Kanagawa 210-0821 (JP)
(74) Representative: van Dam, Vincent
(86) International application number: PCT/JP2022/000356
(87) International publication number: WO 2022/149610

(57) **Abstract**

[Problem] To provide a guanidine derivative (in particular, an amino acid compound having a 4-guanidino group) which has a low production cost and is less likely to cause the problem of waste liquor, a method for producing the same and an intermediate to be used in the production of the same.

[Solution] A compound represented by formula (I), a tautomer thereof, an enantiomer thereof, a pharmaceutically acceptable salt thereof or a pharmaceutically acceptable solvate thereof; a method for producing a compound represented by formula (I), a tautomer thereof, an enantiomer thereof, a pharmaceutically acceptable salt thereof or a pharmaceutically acceptable solvate thereof, said method comprising a mixing step for mixing a compound represented by formula (II) with a compound represented by formula (III); and a compound represented by formula (IV) or formula (V).

## Description

### TECHNICAL FIELD

The present invention relates to a novel guanidine derivative and others.

### BACKGROUND ART

A guanidino group is known to form a hydrogen bond with, for example, a sulfate group, a carboxy group, or a phosphate group by electrostatic interactions. Arginine is known as a natural amino acid having a guanidino group, and the guanidino group of arginine is known to play an important role in interaction with cell membranes, cell entry, or the like in a living body. On the other hand, because arginine is a natural amino acid, it is prone to be degraded by a protease or the like. Therefore, if arginine is to be used as a medicament, issues concerning blood half-life or the like are apt to occur.

4-Guanidinophenylalanine (F4G) is known as an alternative to arginine (J. Med. Chem 2012, 55, 10287-10291). This compound is resistant to trypsin (Chem. Pharm. Bull. 1986, 34(3),1351-1354), and has a high stability (J. Med. Chem. 2012, 55, 6294-6305), and thus is one of the amino acid derivatives that are emphasized in peptide pharmaceuticals. Presently, as a method for synthesis of F4G, a method using mercury or silver is known (Tetrahedron Letters 1996, 37, 48, 8711-8714) . However, since the method uses heavy metals, it is costly to produce F4G, and waste solvents are problematic.

### PRIOR ART DOCUMENTS

### NON-PATENT DOCUMENTS

Non-patent document 1: J. Med. Chem. 2012, 55, 10287-10291
Non-patent document 2: Chem. Pharm. Bull. 1986, 34(3), 1351-1354
Non-patent document 3: J. Med. Chem. 2012, 55, 6294-6305
Non-patent document 4: Tetrahedron Letters 1996, 37, 48, 8711-8714

### SUMMARY OF THE INVENTION

### PROBLEMS TO BE SOLVED BY THE INVENTION

An object of the present invention is to provide a guanidine derivative (in particular, an amino acid compound having a 4-guanidino group) that is produced at a low cost and with a low probability of occurrence of waste solvent problems, a method for manufacture of the derivative, and an intermediate used for the manufacture of the derivative.

### MEANS FOR SOLVING THE PROBLEMS

A first aspect relates to a compound represented by the following Formula (I) (a novel guanidine compound), a tautomer thereof, an enantiomer thereof, a pharmaceutically acceptable salt thereof, or a pharmaceutically acceptable solvate thereof. 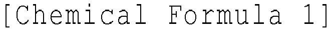 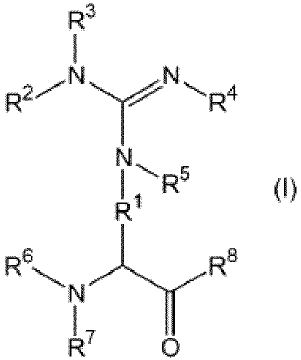 in Formula (1),
-R¹- represents a C₁-C₁₀ alkylene group which may have one or more substituent groups selected from the group A, a C₆₋₁₀ arylene group which may have one or more substituent groups selected from the group A, or a group represented by -R¹¹-R¹²-,
the group A consists of a halogen atom, an amino group, a nitro group, a C₁₋₅ alkyl group, a C₁₋₃ alkoxy group, a C₁₋₃ alkylthio group, and a C₁₋₃ halogenoalkyl group,
-R¹¹- represents a C₆₋₁₀ arylene group which may have one or more substituent groups selected from the group A,
the group represented by -R¹²- represents a C₁-C₄ alkylene group which may have one or more substituent groups selected from the group A,
R² and R⁴ may be the same or different and represent a protecting group or a hydrogen atom,
R³ represents a hydrogen atom,
R⁵ represents a hydrogen atom, a halogen atom, a hydroxy group, an amino group, a nitro group, a C₁₋₅ alkyl group, a C₁₋₃ alkoxy group, a C₁₋₃ alkylthio group, or a C₁₋₃ halogenoalkyl group, or R⁵ is taken together with -N-R¹- to form a C₅₋₇ heterocyclic amine which may have an asymmetric carbon atom at the alpha-position,
R⁶ and R⁷ may be the same or different and represent a protecting group, a C₁₋₅ alkyl group or a hydrogen atom,
R⁸ represents a protecting group of an amino acid, a hydrogen atom, a halogen atom, a hydroxy group, an amino group, a nitro group, a C₁₋₅ alkyl group, a C₁₋₃ alkoxy group, a C₁₋₃ alkylthio group, a C₁₋₃ halogenoalkyl group or a group represented by -O-R¹³-, and
the group represented by R¹³- represents a protecting group of an oxygen atom, a C₁₋₅ alkyl group, a C₁₋₃ alkoxy group, a C₁₋₃ alkylthio group, or a C₁₋₃ halogenoalkyl group,
with the proviso that a compound in which -R¹ represents a propylene group, R² to R⁷ each represent a hydrogen atom, and R⁸ represents a hydroxy group, and
a compound in which -R¹- represents a group represented by -R¹¹-R¹²-, -R¹¹- represents a 1,4-phenylene group, -R¹²- represents a methylene group, R², R⁴, R⁵, R⁶, and R⁷ each represents a hydrogen atom, and R⁸ represents a hydroxy group are excluded.

A second aspect relates to a method for manufacture of a peptide using the above-described compound, a tautomer thereof, an enantiomer thereof, a pharmaceutically acceptable salt thereof, or a pharmaceutically acceptable solvate thereof.

A third aspect relates to a method for manufacture of a compound represented by Formula (I), a tautomer thereof, an enantiomer thereof, a pharmaceutically acceptable salt thereof, or a pharmaceutically acceptable solvate thereof, the method including a mixing step in which a compound represented by the following Formula (II) is mixed with a compound represented by the following Formula (III). 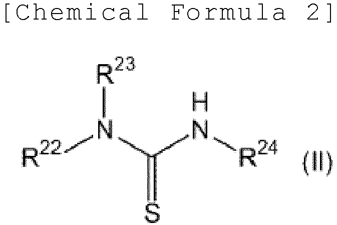
(R²², R²³ and R²⁴ have the same meaning as R², R³ and R⁴, respectively.) 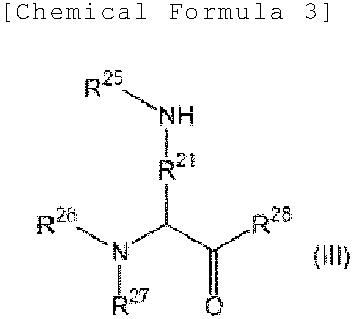
(R²¹, R²⁵, R²⁶, and R²⁷ have the same meaning as R¹, R⁵, R⁶, and R⁷, respectively,
R²⁸ represents a group represented by -O-R³³-, and
the group represented by R³³- represents a protecting group of an oxygen atom.)

### EFFECT OF THE INVENTION

According to the present invention, by using a novel thiourea compound as an intermediate, a guanidine derivative (in particular, an amino acid compound having a 4-guanidino group) can be obtained at a low cost and with a low probability of occurrence of waste fluid problems.

### EMBODIMENTS FOR CARRYING OUT THE INVENTION

Hereinafter, embodiments for carrying out the present invention will be described. The present invention is not limited to the embodiments described below, and includes those that are properly modified from the following embodiments and obvious to one of ordinary skill in the art.

### Guanidine derivative

A novel guanidine derivative of the present invention relates to a compound represented by the following Formula (I), a tautomer thereof, an enantiomer thereof, a pharmaceutically acceptable salt thereof, or a pharmaceutically acceptable solvate thereof.

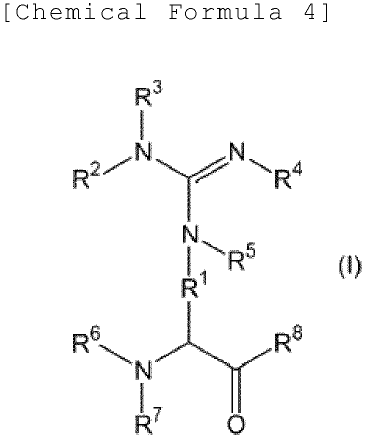

-R¹- represents a C₁-C₁₀ alkylene group which may have one or more substituent groups selected from the group A, a C₆₋₁₀ arylene group which may have one or more substituent groups selected from the group A, or a group represented by -R¹¹-R¹²-.

The group A consists of a halogen atom, an amino group, a nitro group, a C₁₋₅ alkyl group, a C₁₋₃ alkoxy group, a C₁₋₃ alkylthio group, and a C₁₋₃ halogenoalkyl group.

-R¹¹- represents a C₆₋₁₀ arylene group which may have one or more substituent groups selected from the group A.
the group represented by -R¹²- represents a C₁-C₄ alkylene group which may have one or more substituent groups selected from the group A.

R² and R⁴ may be the same or different and represent a protecting group or a hydrogen atom, and R³ represents a hydrogen atom.

R⁵ represents a hydrogen atom, a halogen atom, a hydroxy group, an amino group, a nitro group, a C₁₋₅ alkyl group, a C₁₋₃ alkoxy group, a C₁₋₃ alkylthio group, or a C₁₋₃ halogenoalkyl group. R⁵ may be taken together with -N-R¹- to form a C₅₋₇ heterocyclic amine which may have an asymmetric carbon atom at the alpha-position.

R⁶ and R⁷ may be the same or different and represent a protecting group, a C₁₋₅ alkyl group or a hydrogen atom.

R⁸ represents a hydrogen atom, a halogen atom, a hydroxy group, an amino group, a nitro group, a C₁₋₅ alkyl group, a C₁₋₃ alkoxy group, a C₁₋₃ alkylthio group, a C₁₋₃ halogenoalkyl group or a group represented by-O-R¹³-. R⁸ may be a protecting group of an amino acid.

The group represented by R¹³- represents a protecting group of an oxygen atom, a C₁₋₅ alkyl group, a C₁₋₃ alkoxy group, a C₁₋₃ alkylthio group, or a C₁₋₃ halogenoalkyl group.

However, among the compounds represented by Formula (I), a compound in which -R¹ represents a propylene group, R² to R⁷ each represent a hydrogen atom, and R⁸ represents a hydroxy group (arginine), and
a compound in which -R¹- represents a group represented by -R²¹-R¹²-, -R¹¹- represents a 1,4-phenylene group, -R¹²- represents a methylene group, R², R⁴, R⁵, R⁶, and R⁷ each represents a hydrogen atom, and R⁸ represents a hydroxy group (F4G) are excluded from the guanidine derivatives of the present invention.

The tautomers thereof refer to tautomers of a compound represented by Formula (I). The tautomers refer to isomers in which the isomers are rapidly converted to each other, i.e., isomerized, and an equilibrium state in which both of the isomers coexist can be achieved.

The enantiomers thereof refer to enantiomers of a compound represented by Formula (I). The enantiomers refer to isomers in which one of the two molecules is a chiral molecule (a mirror image) of the other, and the molecule cannot be superposed on the other and vice versa.

The pharmaceutically acceptable salt thereof refers to a pharmaceutically acceptable salt of a compound represented by Formula (I). Examples of the pharmacologically acceptable salt include salts formed with an inorganic base, ammonia, an organic base, an inorganic acid, an organic acid, a basic amino acid, a halogen ion, and the like, and an inner salt. Examples of the inorganic base include an alkali metal (Na, K, etc) and an alkaline earth metal (Ca, Mg, etc). Examples of the organic base include trimethylamine, triethylamine, choline, procaine, ethanolamine, dicyclohexyl amine, cyclohexyl amine, cinchonidine, pyridine, lutidine, triethylenediamine, and the like. Examples of the inorganic acid include hydrochloric acid, hydrobromic acid, sulfuric acid, nitric acid, and phosphoric acid, and the like. Examples of the organic acid include p-toluenesulfonic acid, methanesulfonic acid, formic acid, trifluoroacetic acid, and maleic acid, and the like. Examples of the basic amino acid include lysine, arginine, ornithine, histidine, and the like.

The pharmaceutically acceptable solvate thereof refers to a pharmaceutically acceptable solvate of a compound represented by Formula (I). An example of the solvate is a hydrate. A compound of the present invention sometimes absorbs moisture present in the air during storage, and forms a hydrate. The present invention embraces such a hydrate. The pharmaceutically acceptable solvate thereof may be a solvate of a pharmaceutically acceptable salt of a compound represented by Formula (I).

Preferred examples of the compound represented by Formula (I) are those in which -R¹- represents a group represented by -R¹¹-R¹²-.
In the examples, -R¹¹- represents a 1,3-phenylene group or a 1,4-phenylene group which may have one or more substituent groups selected from the group A, and the group represented by -R¹²- represents a C₁₋₄ alkylene group.

In the examples, more preferred are those in which -R¹¹- represents a 1,3-phenylene group or a 1,4-phenylene group, and the group represented by -R¹²-represents a C₁₋₄ alkylene group.

In the examples described above, those in which R⁵, R⁶, R⁷, and R⁸ represent the following groups are preferred.

R⁵ represents a hydrogen atom, a methyl group, or an ethyl group.

R⁶ represents an Fmoc group (9-fluorenylmethyloxycarbonyl group).

R⁷ represents a hydrogen atom.

R⁸ represents a hydroxy group, or a group represented by -O-R¹³-, wherein
the group represented by R¹³- represents a protecting group of an oxygen atom, a methyl group, or an ethyl group.

Preferred examples of the compound represented by Formula (I) other than those described above are those in which -R¹- represents a C₃-C₆ alkylene group. Among these, those in which -R¹- represents a C₄ alkylene group are preferred examples. Preferred examples of the compound represented by Formula (I) are those in which -R¹- represents a C₄ alkylene group, R² represents a protecting group (e.g., Rbf-), R³ represents a hydrogen atom, R⁴ represents a protecting group (e.g., Sub-), R⁵ represents a methyl group, R⁶ represents an Fmoc group, R⁷ represents a hydrogen atom, R⁸ represents a hydroxy group (e.g., N2-(((9H-fluoren-9-yl)methoxy)carbonyl)-N6-(N'-((10,11-dihydro-5H-dibenzo[a,d][7]annulene-5-yl)-N-((2,2,4,6,7-pentamethyl-2,3-dihydrobenzofuran-5-yl)sulfonyl)carbamimidoyl)-N6-methyl-L-lysine).

In those in which -R¹- in Formula (I) represents a C₃-C₆ alkylene group, those in which R⁵, R⁶, R⁷, and R⁸ represent the following groups are preferred.

R⁵ represents a hydrogen atom, a methyl group, or an ethyl group.

R⁶ represents an Fmoc group (9-fluorenylmethyloxycarbonyl group).

R⁷ represents a hydrogen atom, a methyl group, or an ethyl group.

R⁸ represents a hydroxy group, or a group represented by -O-R¹³-, wherein
the group represented by R¹³- represents a protecting group of an oxygen atom, a methyl group, or an ethyl group.

Examples of the compound in which R⁵ is taken together with -N-R¹- to form a C₅₋₇ heterocyclic amine which may have an asymmetric carbon atom at the alpha-position are those described below.

### [Chemical Formula 5]

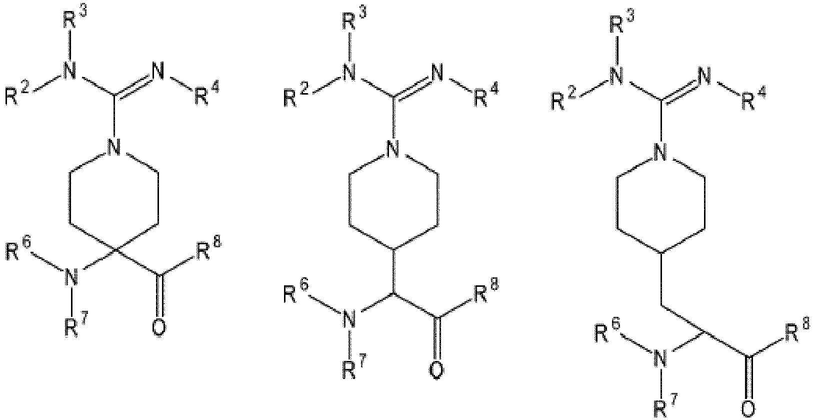

In the compound represented by Formula (I), preferred examples other than those described above are compounds in which R⁷ is a hydrogen atom, R⁸ is a protecting group (a protecting group of an amino acid), a C₁₋₅ alkyl group or a hydrogen atom (i.e., a compound being a peptoid and having a piperazine ring).

Herein, "Cₘ₋ₙ" means that the number of carbon atoms is any one of from m to n.

An alkyl group refers to a monovalent group which is formed by removing one hydrogen atom from a linear or branched aliphatic hydrocarbon. Examples of a C₁-₅ alkyl group are a methyl group, an ethyl group, a propyl group, an isopropyl group, a butyl group, an isobutyl group, a sec-butyl group, a tert-butyl group, a pentyl group, an iso-pentyl group, and a neopentyl group. Examples of a C₁-C₄ alkyl group are a methyl group, an ethyl group, a propyl group, an isopropyl group, a butyl group, an isobutyl group, a sec-butyl group, and a tert-butyl group.

An alkoxy group refers to a monovalent group which is formed by removing a hydrogen atom from a hydroxyl group of a linear or branched alcohol. Examples of a C₁₋₃ alkoxy group are a methoxy group, an ethoxy group, a propoxy group, and an iso-propoxy group.

An alkylthio group refers to a group in which an oxygen atom of an alkoxy group is substituted by a sulfur atom. Examples of a C₁₋₃ alkylthio group are a methylthio group, an ethylthio group, a propylthio group, and an isopropylthio group.

A halogenoalkyl group refers to a monovalent group in which one or more hydrogen atoms of an alkyl group are substituted by one or more halogen atoms. Examples of a C₁₋₃ halogenoalkyl group are a trifluoromethyl group, a trichloromethyl group, a difluoromethyl group, a dichloromethyl group, a dibromomethyl group, a fluoromethyl group, a 2,2,2-trifluoroethyl group, a 2,2,2-trichloroethyl group, a 2-bromoethyl group, a 2-chloroethyl group, a 2-fluoroethyl group, a 2-iodoethyl group, a 3-chloropropyl group, and a 4-fluorobutyl group.

An alkylene group refers to a divalent group which is formed by removing two hydrogen atoms from a linear or branched aliphatic hydrocarbon. Examples of a C₁₋₁₀ alkylene group are a methylene group, a methylmethylene group, an ethylene group, a propylene group, a trimethylene group, a 1-methylethylene group, a tetramethylene group, a 1-methyltrimethylene group, a 2-methyltrimethylene group, a 3-methyltrimethylene group, a 1-methylpropylene group, a 1,1-dimethylethylene group, a pentamethylene group, a 1-methyltetramethylene group, a 2-methyltetramethylene group, a 3-methyltetramethylene group, a 4-methyltetramethylene group, a 1,1-dimethyltrimethylene group, a 2,2-dimethyltrimethylene group, a 3,3-dimethyltrimethylene group, a hexamethylene group, a 1-methylpentamethylene group, a 2-methylpentamethylene group, a 3-methylpentamethylene group, a 4-methylpentamethylene group, a 5-methylpentamethylene group, a 1,1-dimethyltetramethylene group, a 2,2-dimethyltetramethylene group, a 3,3-dimethyltetramethylene group, a 4,4-dimethyltetramethylene group, a heptamethylene group, a 1-methylhexamethylene group, a 2-methylhexamethylene group, a 5-methylhexamethylene group, a 3-ethylpentamethylene group, an octamethylene group, a 2-methylheptamethylene group, a 5-methylheptamethylene group, a 2-ethylhexamethylene group, a 2-ethyl-3-methylpentamethylene group, and a 3-ethyl-2-methylpentamethylene group. The alkylene group is preferably a C₁₋₄ alkylene group, and more preferably a C₁₋₂ alkylene group.

An arylene group refers to a divalent group which is formed from an aromatic hydrocarbon by removing two hydrogen atoms bonded to the ring. Examples of the ring that constitutes a C₆₋₁₀ arylene group are a benzene ring or a naphthalene ring.

Examples of halogen atoms are fluorine, chlorine, bromine, and iodine.

Examples of the protecting group are 10,11-dihydro-5H-dibenzo-[a,d][7]annulene-5-yl group (Sub), a diphenylmethyl group (Bzh), a 4-methoxyphenylmethyl group (PMB), a tert-butyl group (tBu), a tert-butoxycarbonyl group (Boc), a 9-fluorenylmethyloxycarbonyl group (Fmoc), a tosyl group (Tos), a nitro group (NO₂), a 4-methoxy-2,3,6-trimethylbenzenesulfonyl group (Mtr), a 2,2,5,7,8-pentamethylchroman-6-sulfonyl group (Pmc), a 2,2,4,6,7-pentamethyl-2,3-dihydrobenzofuran-5-sulfonyl group (Pbf), benzyloxycarbonyl group (Z), a 2-chlorobenzyloxycarbonyl group (Cl-Z), a 3-nitro-2-pyridinesulfenyl group (Npys), a phenacyl group (Pac), a benzyloxymethyl group (Bom), a dinitrophenyl group (Dmp), a trityl group (Trt), a benzyl group (Bzl), a 4-methoxybenzyl group (MBzl), a 4-methylbenzyl group (4-MeBzl), a acetamidomethyl group (Acm), a tert-butylthio group (tBuS), a 2,6-dichlorobenzyl group (Cl₂-Bzl), a formyl group (CHO), a benzyl ester group (OBzl), a tert-butyl ester group (OtBu), a cyclohexyl ester group (OcHex), a phenacyl ester group (OPac), and a xanthyl group (Xan).

### Method for manufacture of peptides

Next, a method for manufacture of peptides using the above-described compound, a tautomer thereof, an enantiomer thereof, a pharmaceutically acceptable salt thereof, or a pharmaceutically acceptable solvate thereof will be described. The guanidine derivative of the present invention has a guanidino group, and thus peptides and proteins can be synthesized by joining the guanidino group. Furthermore, the above-described compound and the like and an amino acid can be mixed together and reacted to obtain a peptide.

### Thiourea compound

The novel thiourea compound as an intermediate used for manufacture of the guanidine derivative is a compound represented by the following Formula (II).

### [Chemical Formula 6]

R²² and R²⁴ may be the same or different and represent a protecting group or a hydrogen atom. Examples of the protecting group are Pbf, Pmc or Sub. Among these, preferred are those in which R²² and R²⁴ are Pbf and Sub, respectively.
R²³ represents a hydrogen atom.

Specific examples of the compound represented by Formula (II) are compounds represented by the following Formula (VI) or (V).

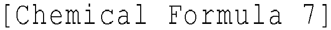

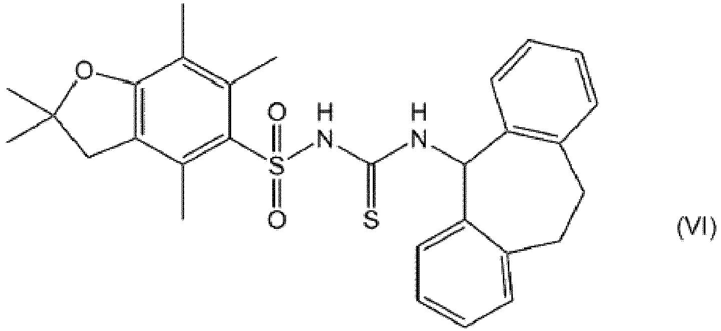

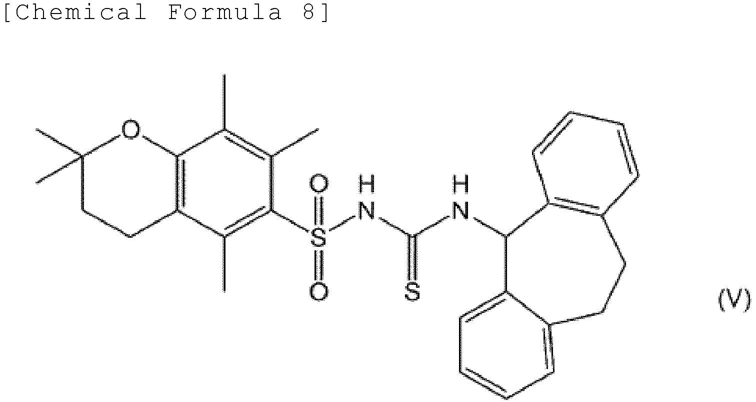

Specific compounds represented by Formula (I) are as follows:
i. 2-((((9H-fluoren-9-yl)methoxy)carbonyl)amino)-3-(4-(2-(10,11-dihydro-5H-dibenzo[a,d][7]annulene-5-yl)-3-((2,2,4,6,7-pentamethyl-2,3-dihydrobenzofuran-5-yl)sulfonyl)guanidino)phenyl)propanoic acid;
ii. 2-((((9H-fluoren-9-yl)methoxy)carbonyl)amino)-3-(3-(2-(10,11-dihydro-5H-dibenzo[a,d][7]annulene-5-yl)-3-((2,2,4,6,7-pentamethyl-2,3-dihydrobenzofuran-5-yl)sulfonyl)guanidino)phenyl)propanoic acid;
iii. N2-(((9H-fluoren-9-yl)methoxy)carbonyl)-N6-(N'-(10,11-dihydro-5H-dibenzo[a,d][7]annulene-5-yl)-N-((2,2,4,6,7-pentamethyl-2,3-dihydrobenzofuran-5-yl)sulfonyl)carbamimidoyl-L-lysine;
iv. N-(((9H-fluoren-9-yl)methoxy)carbonyl)-N-(3-(3-(10,11-dihydro-5H-dibenzo[a,d][7]annulene-5-yl)-2-((2,2,4,6,7-pentamethyl-2,3-dihydrobenzofuran-5-yl)sulfonyl) (guanidino)propyl)glycine;
v. 2-((((9H-fluoren-9-yl)methoxy)carbonyl)amino)-3-(1-(N'-(10,11-dihydro-5H-dibenzo[a,d][7]annulene-5-yl)-N-((2,2,4,6,7-pentamethyl-2,3-dihydrobenzofuran-5-yl)sulfonyl)carbamimidoyl)piperidine-4-yl)propanoic acid;
vi. N2-(((9H-fluoren-9-yl)methoxy)carbonyl)-N6-(N'-(10,11-dihydro-5H-dibenzo[a,d][7]annulene-5-yl)-N-((2,2,4,6,7-pentamethyl-2,3-dihydrobenzofuran-5-yl)sulfonyl)carbamimidoyl)-N6-methyl-L-lysine;
vii. N2-(((9H-fluoren-9-yl)methoxy)carbonyl)-Nw'-(10,11-dihydro-5H-dibenzo[a,d][7]annulene-5-yl)-Nd-methyl-Nw-((2,2,4,6,7-pentamethyl-2,3-dihydrobenzofuran-5-yl)sulfonyl)-L-arginine;
viii. 4-((((9H-fluoren-9-yl)methoxy)carbonyl)amino)-1-(N'-(10,11-dihydro-5H-dibenzo[a,d][7]annulene-5-yl)-N-((2,2,4,6,7-pentamethyl-2,3-dihydrobenzofuran-5-yl)sulfonyl)carbamimidoyl)piperidine-4-carboxylic acid;
ix. 2-((((9H-fluoren-9-yl)methoxy)carbonyl)amino)-2-(1-(N'-(10,11-dihydro-5H-dibenzo[a,d][7]annulene-5-yl)-N-((2,2,4,6,7-pentamethyl-2,3-dihydrobenzofuran-5-yl)sulfonyl)carbamimidoyl)piperidine-4-yl)acetic acid;
x. 2-((((9H-fluoren-9-yl)methoxy)carbonyl)amino)-4-(2-(10,11-dihydro-5H-dibenzo[a,d][7]annulene-5-yl)-3-((2,2,4,6,7-pentamethyl-2,3-dihydrobenzofuran-5-yl)sulfonyl)guanidino)butanoic acid;
xi. N2-(((9H-fluoren-9-yl)methoxy)carbonyl)-N6-(N-(10,11-dihydro-5H-dibenzo[a,d][7]annulene-5-yl)-N'-((2,2,4,6,7-pentamethyl-2,3-dihydrobenzofuran-5-yl)sulfonyl)carbamimidoyl)-N6-methyl-D-lysine;
xii. 2-((((9H-fluoren-9-yl)methoxy)carbonyl)amino)-3-(4-(3-(10,11-dihydro-5H-dibenzo[a,d][7]annulene-5-yl)-2-((2,2,4,6,7-pentamethyl-2,3-dihydrobenzofuran-5-yl)sulfonyl)guanidinophenyl)propanoic acid;
xiii. 2-((((9H-fluoren-9-yl)methoxy)carbonyl)amino)-3-(3-(3-(10,11-dihydro-5H-dibenzo[a,d][7]annulene-5-yl)-2-((2,2,4,6,7-pentamethyl-2,3-dihydrobenzofuran-5-yl)sulfonyl)guanidino)phenyl)propanoic acid; and
xiv. N2-(((9H-fluoren-9-yl)methoxy)carbonyl)-Nw-(10,11-dihydro-5H-dibenzo[a,d][7]annulene-5-yl)-Nd-methyl-Nw'-((2,2,4,6,7-pentamethyl-2,3-dihydrobenzofuran-5-yl)sulfonyl)-D-arginine.

### Synthesis of thiourea compounds

The novel thiourea compounds of the present invention can be manufactured according to the following scheme.

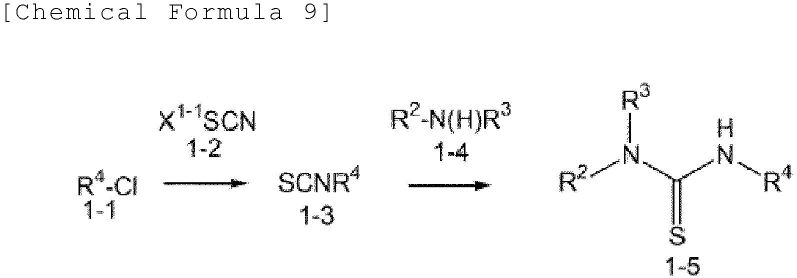

R² and R⁴ may be the same or different and represent a protecting group or a hydrogen atom. Indeed, in Formula 1-1, Formula 1-3, Formula 1-4, those in which R² and R⁴ each represent a protecting group are preferred. Examples of R² and R⁴ are Pbf and Sub, respectively.

R³ represents a hydrogen atom. In Formula 1-2, X¹⁻¹ represents an alkali metal (e.g., Na, K) or an alkaline earth metal (the compositional formula of X¹⁻¹SCN varies depending on the valency of a cation).

A compound represented by Formula 1-1 is dissolved in an organic solvent (e.g., N,N-dimethylformamide: DMF). To this solution is added a compound represented by Formula 1-2, and the solution is stirred to obtain a solution containing a compound represented by Formula 1-3. The molar ratio of compounds represented by formula 1-1 and formula 1-2 may be 1:2 to 2:1, 2:3 to 3:2, or 4:5 to 5:4. The reaction temperature may be 10°C or higher and 60°C or lower, or 20°C or higher and 40°C or lower. The reaction time may be 10 minutes or more and 3 hours or less, or 20 minutes or more and 1 hour or less. A known catalyst may be added to the reaction system. The stirring speed may be properly adjusted.

A compound represented by Formula 1-4 is dissolved in an organic solvent (e.g., DMF). To this solution is added a strong base (e.g., NaH) with cooling. The resulting solution with the added strong base is stirred to obtain a reaction solution (e.g., suspension).

The molar ratio of compounds represented by Formula 1-1 and Formula 1-4 may be 1:2 to 2:1, 2:3 to 3:2, or 4:5 to 5:4. The temperature when a strong base is added to the compound represented by Formula 1-4 is -20°C or higher and 10°C or lower, and may be -10°C or higher and 5°C or lower. The temperature of the solution while stirring may be 10°C or higher and 60°C or lower, or 20°C or higher and 40°C or lower. The reaction time may be 5 minutes or more and 2 hours or less, or 10 minutes or more and 1 hour or less. A known catalyst may be added to the reaction system. The stirring speed may be properly adjusted. Consequently, a solution containing a compound represented by Formula 1-4 is obtained.

A solution containing a compound represented by Formula 1-3 is added to the solution containing a compound represented by Formula 1-4, and the resulting solution is stirred to promote a reaction and obtain a solution containing a compound represented by Formula 1-5. The temperature of the solution while stirring may be 10°C or higher and 60°C or lower, or 20°C or higher and 40°C or lower. The reaction time may be 10 minutes or more and 4 hours or less, or 30 minutes or more and 2 hours or less.

To quench this reaction, it is adequate to cool the solution, and add a strong acid (e.g., hydrochloric acid) to the solution. After quenching the reaction, the resulting reaction solution can be purified and dried using known methods for purification and/or drying to obtain a compound represented by Formula 1-5.

### Synthesis of guanidine derivative (1)

The guanidine derivative of the present invention can be manufactured according to the following scheme. This step utilizes a reaction in which when the C-terminal of an amino acid of a side chain is not protected, a protecting group derived from a silylation reagent protects the unprotected C-terminal of the amino acid of the side chain.

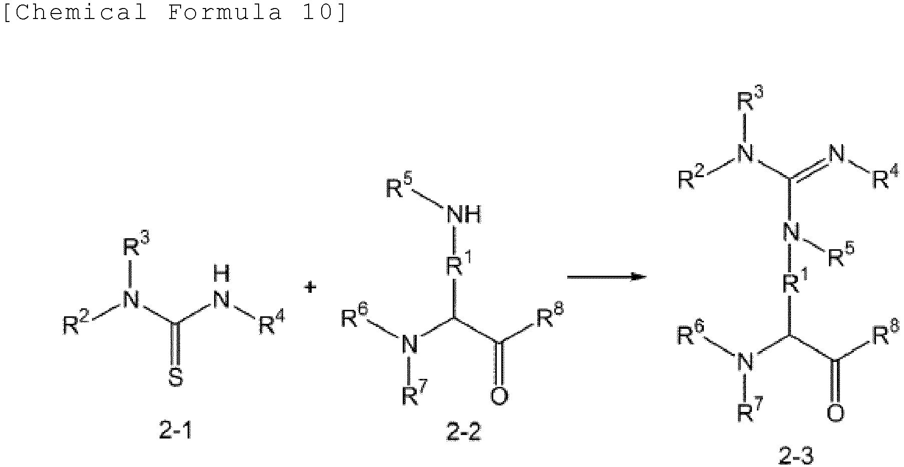

R¹ to R⁸ have the same meaning as provided above. A compound represented by Formula 2-2 is mixed with an organic solvent (e.g., tetrahydrofuran: THF), and in particular when the C-terminal is not protected (i.e., when R⁸ is a hydroxy group), a silylation reagent (e.g., MSA) is added and the resulting mixture is stirred. The molar ratio of the compound represented by Formula 2-2 and the silylation reagent may be 1:4 to 2:1, 1:3 to 1:1, or 1:2 to 1:1. The temperature of the solvent while stirring may be 10°C or higher and 90°C or lower, or 20°C or higher and 80°C or lower. The stirring time may be 20 minutes or more and 3 hours or less, or 30 minutes or more and 2 hours or less.

To a liquid containing the above-described compound represented by Formula 2-2 are added a compound represented by Formula 1-5 and a condensing agent, and the resulting mixture is stirred. An organic base (e.g., TEA) may be added to this reaction system. The condensing agent may be a known condensing agent, and an example is dimethylaminopropylethylcarbodiimide hydrochloride (EDCI). The stirring time may be 10 minutes or more and 2 hours or less, or 20 minutes or more and 1 hour or less. To quench this reaction, it is adequate to add an acid (e.g., citric acid) to the reaction liquid. The molar ratio of compounds represented by Formula 2-1 and Formula 1-5 may be 1:2 to 2:1, 2:3 to 3:2, or 4:5 to 5:4.

Consequently, a liquid containing a compound represented by formula 2-3 can be obtained. From this liquid, a compound represented by formula 2-3 can be obtained by extraction, purification, and/or drying. Note that the extraction may include treatments such as salt formation or purification.

### Synthesis of guanidine derivative (2)

When the group R² or R⁴ in the compound represented by 2-3 is a protecting group, the group can be deprotected to obtain a deprotected guanidine derivative of the present invention.

### Applications of guanidine derivative

A guanidine derivative of the present invention can be used for the following applications.

A guanidine derivative (in particular, a salt) of the present invention easily forms a hydrogen bond, and thus utilized as a denaturant of a protein.

A guanidine derivative of the present invention is utilised as a strong base in organic synthesis.

A guanidine derivative of the present invention has a guanidino group, and thus peptides and proteins can be synthesized by joining the guanidino group. A guanidine derivative or a peptide of the present invention has a cell membrane-permeating property, and thus also has efficacy as a carrier of a drug delivery system.

### [Example 1]

Hereinafter, synthesis examples as Reference Examples, Comparative Examples, and Examples will be provided, and the present invention will be described in further detail, but the present invention is not limited to the Examples.

Herein, when amino acids and others are expressed by abbreviations, each of the expressions is based on an abbreviation according to IUPAC-IUB Commission on Biochemical Nomenclature or abbreviations generally used in the art.

Note that abbreviated expressions in synthesis examples are as follows.
Fmoc as 9-fluorenylmethyloxycarbonyl or 9-fluorenylmethoxycarbonyl;
Pbf as 2,2,4,6,7-pentamethyldihydrobenzofuran-5-sulfonyl;
Pmc as 2,2,5,7,8-pentamethylchroman-6-sulfonyl;
Sub as 10,11-dihydro-5H-dibenzo[A,D][7]annulene-5-yl;
Bzh as benzhydryl;
tBu as tert-butyl;
Pmb as p-methoxybenzyl;
THF as tetrahydrofuran;
MSA as N-methyl-N-trimethylsilylacetamide;
TEA as triethylamine;
EDCI as dimethylaminopropylethylcarbodiimide hydrochloride;
DMF as N,N-dimethylformamide;
DCM as dichloromethane;
TIS as triisopropylsilane;
DODT as 3,6-dioxa-1,8-octane-dithiol;
mL as milliliter (unit);
M as molar (unit: mol/L);
mM as millimolar (unit);
mm as millimeter (unit);
nm as nanometer (unit);
Å as angstrom (unit);
min as minute (unit);
MS as mass spectrometry;
mmol as millimole (unit);
mg as milligram (unit); and
LC-MS or LC/MS as a liquid chromatography-mass spectrometer.

Abbreviated expressions other than those described above are as follows.
F4G as 2-amino-3-(4-guanidinophenyl)propanoic acid;
F3G as 2-amino-3-(3-guanidinophenyl)propanoic acid;
Har as N6-carbamimidoyl-L-lysine;
GpG as (3-guanidinopropyl)glycine;
A4pG as 2-amino-3-(1-carbamimidoylpiperidine-4-yl)propanoic acid;
HarMe as N6-carbamimidoyl-N6-methyl-L-lysine;
RMe as Nd-methyl-L-arginine;
A4pipG as 4-amino-1-carbamimidoylpiperidine-4-carboxylic acid;
G4pipG as 2-amino-2-(1-carbamimidoylpiperidine-4-yl)acetic acid;
Nar as 2-amino-4-guanidinobutanoic acid;
dhar as N6-carbamimidoyl-D-lysine;
dharMe as N6-carbamimidoyl-N6-methyl-D-lysine;
dF4G as (R)-2-amino-3-(4-guanidinophenyl)propanoic acid;
df3G as (R)-2-amino-3-(3-guanidinophenyl)propanoic acid; and
drMe as Nd-methyl-D-arginine.

Proton magnetic resonance (1H-NMR) in the following synthesis example was measured, unless otherwise indicated, by JNM-ECP300 (manufactured by JEOL), JNM-ECX300 (manufactured by JEOL), or Ascend^{™}500 (manufactured by Bruker) in a solvent deuterochloroform or hexadeuterodimethyl sulfoxide, and the chemical shift is expressed as δ value (in ppm) against tetramethylsilane as an internal standard (0.0 ppm).

In the report of NMR spectrum, "s" refers to singlet, "d" refers to doublet, "t" refers to triplet, "q" refers to quartet, "dd" refers to doublet of doublets, "dt" refers to doublet of triplets, "sept" refers to septet, "m" refers to multiplet, "br" refers to broad, "J" refers to a coupling constant, "Hz" refers to Hertz, "CDCl3" refers to deuterochloroform, "DMSO-d6" refers to hexadeuterodimethyl sulfoxide.

High performance liquid chromatography/mass spectrometry was measured, unless otherwise specified, by any of ACQUITY UPLC H-Class/QDa (manufactured by Waters Corporation), ACQUITY UPLC H-Class/SQD2 (manufactured by Waters Corporation), or LC-20AD/Triple Tof5600 (manufactured by SHIMADZU CORPORATION).

In the report of high performance liquid chromatography/mass spectrometry, ESI+ refers to positive mode in electrospray ionization, M+H refers to a proton adduct, and M+Na refers to a sodium adduct.

In the report of high performance liquid chromatography/mass spectrometry, ESI- refers to a negative mode in electrospray ionization, M-H refers to a proton lost form.

The ratio between a raw material and a product was calculated from the area ratio of peaks obtained under the following analysis conditions in high performance liquid chromatography/mass spectrometry, and the structure of the resulting compound was confirmed by 1H-NMR and LC/MS.

### Analysis condition A

Column: Kinetex (registered trademark) EVO C18, 2.6 µm, 2.1 × 50 mm.
Mobile phase A: 0.025% TFA in H2O
Mobile phase B: 0.025% TFA in CH3CN
Column temperature: 60°C
Gradient (%B) : 5-95% over 2.1 min; then 95-95% over 0.75 min, flow rate: 0.6 mL/min
Detection: UV 220 nm.

### Analysis condition B

Column: Kinetex (registered trademark) EVO C18 100 angstrom, 1.7 µm, 2.1 × 50 mm.
Mobile phase A: 0.025% TFA in H2O
Mobile phase B: 0.025% TFA in CH3CN
Column temperature: 60°C
Gradient (%B) : 5-95% over 2.1 min, then 95-95% over 0.75 min, flow rate: 0.6 mL/min
Detection: UV 254 nm.

### Synthesis of Pbf-Sub; N-((10,11-dihydro-5H-dibenzo [a, d] [7] annulene-5-yl) carbamothioyl)-2,2,4,6,7-pentamethyl-2,3-dihydrobenzofuran-5-sulfonamide

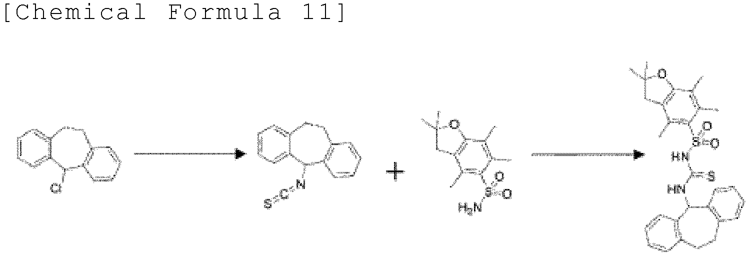

To 5-chloro-10,11-dihydro-5H-dibenzo-[a,d] [7]annulene (22.87 g, 100 mmol) dissolved in 200 mL of DMF was added potassium thiocyanate (10.2 g, 105 mmol), and the mixture was stirred at room temperature for 30 min to obtain a reaction solution. To 2,2,4,6,7-pentamethyl-2,3-dihydrobenzofuran-5-sulfonamide (26.9 g, 100 mmol) dissolved in 200 mL of DMF was added sodium hydride (4.20 g, 105 mmol) at 0°C, and the mixture was stirred at room temperature for 15 min. This suspension was cooled to 0°C, thereafter the reaction solution prepared above was added, and the resulting mixture was stirred at room temperature for 1 hour.

The reaction mixture was cooled to 0°C, thereafter the reaction was quenched with 1 M hydrochloric acid aqueous solution (110 mL), and the reaction mixture was added to 1000 mL of water. The resulting solid was filtered, and the solid was dried at 60°C under reduced pressure overnight. The resulting solid was suspended in ethyl acetate/heptane (1/2, 300 mL), and then the solid was filtered, dried under reduced pressure to obtain the titled compound (42.5 g, 82 mmol, 82% yield). The structure was confirmed by 1H-NMR and LC/MS.

Analysis condition A: retention time=2.25 min, ESI-MS(+) found m/z=521.3 (M+H)+, calculated m/z=520.2

### [Example 2]

### Synthesis of Fmoc-F4G(Pbf,Sub)-OH; (S,Z)-2-((((9H-fluoren-9-yl)methoxy)carbonyl)amino)-3-(4-(2-(10,11-dihydro-5H-dibenzo[a,d][7]annulene-5-yl)-3-((2,2,4,6,7-pentamethyl-2,3-dihydrobenzofuran-5-yl)sulfonyl)guanidino)phenyl)propanoic acid

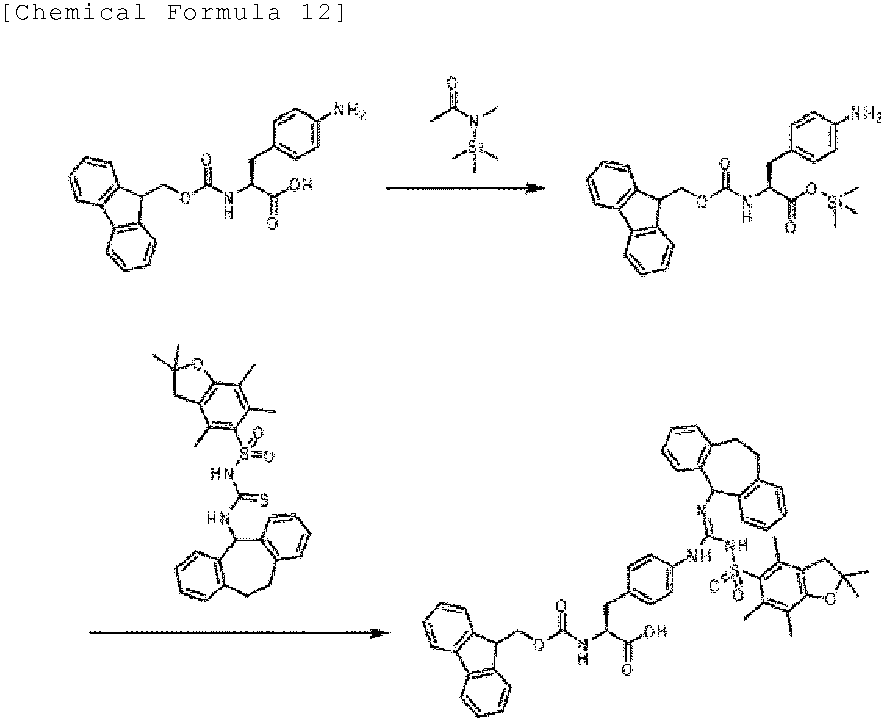

In 7.5 mL of THF, (S)-2-((((9H-fluoren-9-yl)methoxy)carbonyl)amino)-3-(4-aminophenyl)propanoic acid (0.6 g, 1.49 mmol) was suspended, then N-methyl-N-trimethylsilylacetamide (MSA; 0.31 mL, 1.94 mmol) was added, and the mixture was stirred at room temperature for 30 min. To the mixture were added triethylamine (TEA; 0.17 g, 1.64 mmol), N-((10,11-dihydro-5H-dibenzo[a,d][7]annulene-5-yl)carbamothioyl)-2,2,4,6,7-pentamethyl-2,3-dihydrobenzofuran-5-sulfonamide (0.78 g, 1.49 mmol) obtained in Example 1, and 1-(3-dimethylaminopropyl)-3-ethylcarbodiimide hydrochloride (0.286 g, 1.49 mmol), and the mixture was stirred for 60 min. The reaction solution was diluted with 15 mL of ethyl acetate, the reaction was quenched with 1 M sulfuric acid aqueous solution, and then the mixture solution was extracted with ethyl acetate. The resulting organic layer was washed four times with water, then dried with sodium sulfate, and thereafter filtered and concentrated under reduced pressure. The resulting residue was dissolved in DCM (5 mL), and heptane (30 mL) was added to the solution and stirred for 18 hours. The resulting pale-yellow solids were filtered, and the solids were washed with heptane to obtain the titled compound. The structure was confirmed by 1H-NMR and LC/MS.

Analysis condition A: retention time=2.24 min, ESI-MS(+) found m/z=889.5 (M+H)+, calculated m/z=888.4

### [Example 3]

Influence of types of the protecting group on the deprotection efficiency was studied.

### (1) Deprotection of Pbf,Sub of Fmoc-F4G(Pbf,Sub)-Met; methyl (S,Z)-2-((((9H-fluoren-9-yl)methoxy)carbonyl)amino)-3-(4-(2-(10,11-dihydro-5H-dibenzo[a,d][7]annulene-5-yl)-3-((2,2,4,6,7-pentamethyl-2,3-dihydrobenzofuran-5-yl)sulfonyl)guanidino)phenyl)propanoate

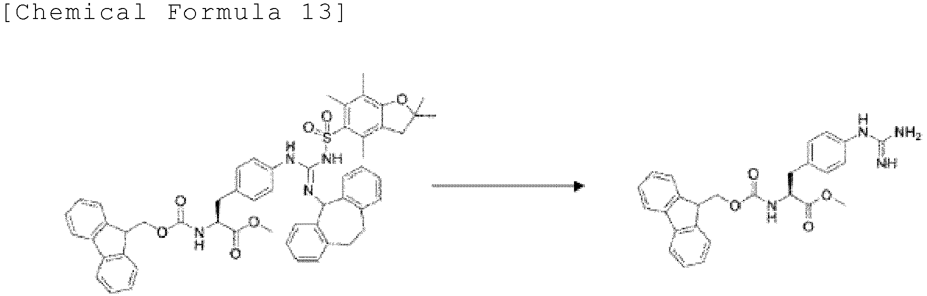

The titled compound was obtained by the same method as in Example 2, except that N-Fmoc-4-amino-L-phenylalaninemethyl; methyl (S)-2-((((9H-fluoren-9-yl)methoxy)carbonyl)amino)-3-(4-aminophenyl)propanoate was used and the MSA treatment was omitted. The resulting compound was added to TFA/TIS/DODT/H2O=92.5:2.5:2.5:2.5, and the mixture was stirred at room temperature for 60 min. The deprotection state was confirmed by LC/MS. Fmoc-F4G-Met; methyl (S)-2-((((9H-fluoren-9-yl)methoxy)carbonyl)amino)-3-(4-guanidinophenyl)propanoate was produced (79.9% in the area of LC/MS).

Analysis conditions for Fmoc-F4G-Met; methyl (S)-2-((((9H-fluoren-9-yl)methoxy)carbonyl)amino)-3-(4-guanidinophenyl)propanoate:
LC/MS conditions were the same as in Example 2.
Retention time=1.35 min, ESI-MS(+) found m/z=459.3 (M+H)+, calculated m/z=458.2

### (2) Synthesis and deprotection of Fmoc-F4G(Pbf,tBu)-OH; (S,Z)-2-((((9H-fluoren-9-yl)methoxy)carbonyl)amino)-3-(4-(2-(tert-butyl)-3-((2,2,4,6,7-pentamethyl-2,3-dihydrobenzofuran-5-yl)sulfonyl)guanidino)phenyl)propanoic acid

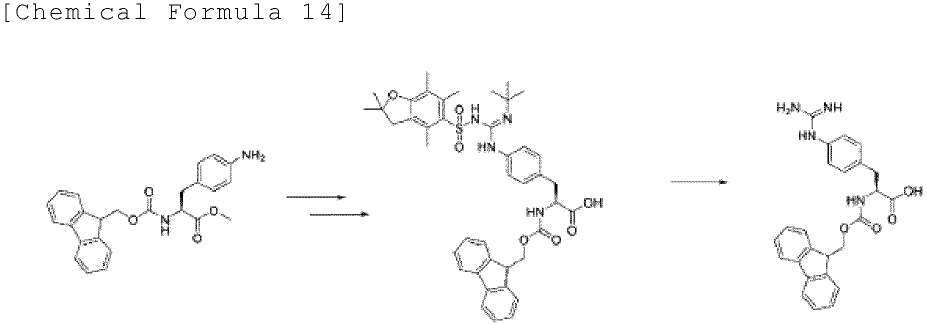

The titled compound was obtained by synthesis and purification according to known methods. The product was confirmed by 1H-NMR.

The resulting compound was deprotected by the same method as in (1). The deprotection treatment was performed for 60 min, and then production of Fmoc-F4G-OH; (S)-2-((((9H-fluoren-9-yl)methoxy)carbonyl)amino)-3-(4-guanidinophenyl)propanoic acid was confirmed by LC/MS (Analysis condition A). As a result, the peak of Fmoc-F4G-OH was scarcely observed, with 4.61% of the total areas (areas in LC/MS), and a peak that represents almost the same mass (found m/z=501.6) as that of Fmoc F4G(H,tBu)-OH; (S,E)-2-((((9H-fluoren-9-yl)methoxy)carbonyl)amino)-3-(4-(2-(tert-butyl)guanidino)phenyl)propanoic acid in which tBu was not deprotected was observed to have an area of 76.8% in LC/MS. Accordingly, it was found that the complete deprotection did not occur.

### (3) Synthesis and deprotection of Fmoc-F4G(Pbf,PMB)-Met; methyl (S,Z)-2-((((9H-fluoren-9-yl)methoxy)carbonyl)amino)-3-(4-(2-(4-methoxybenzyl)-3-((2,2,4,6,7-pentamethyl-2,3-dihydrobenzofuran-5-yl)sulfonyl)guanidino)phenyl)propanoate

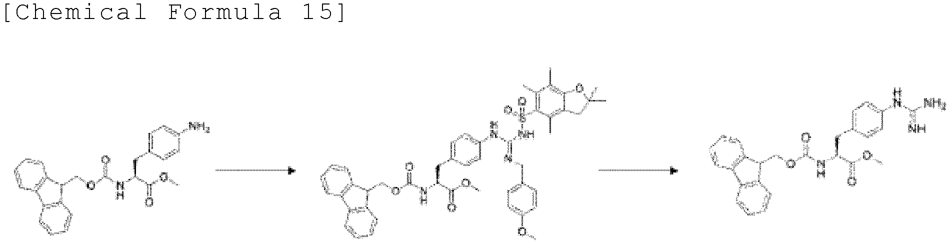

The titled compound was obtained by synthesis and purification according to known methods. The product was confirmed by 1H-NMR.

The resulting compound was deprotected by the same method as in (1). The deprotection treatment was performed for 60 min, and then production of Fmoc-F4G-Met; methyl (S)-2-((((9H-fluoren-9-yl)methoxy)carbonyl)amino)-3-(4-guanidinophenyl)propanoate was confirmed by LC/MS (Analysis condition A). As a result, the peak of Fmoc-F4G-Met was scarcely observed, with 1.32% of the total areas (areas in LC/MS), while a peak of the titled compound in which any deprotection did not occur had an area of 14.3% in LC/MS, and a peak that represents almost the same mass (found m/z=579.6) as that of Fmoc-F4G(H,PMB)-Met; methyl (S,E)-2-((((9H-fluoren-9-yl)methoxy)carbonyl)amino)-3-(4-(2-(4-methoxybenzyl)guanidino)phenyl)propanoate in which PMB was not deprotected remained and had an area of 71.6% in LC/MS. Accordingly, it was found that the complete deprotection did not occur.

### (4) Synthesis and deprotection of Fmoc-F4G(Pbf,Bzh)-Met; methyl (S,Z)-2-((((9H-fluoren-9-yl)methoxy)carbonyl)amino)-3-(4-(2-benzhydryl-3-((2,2,4,6,7-pentamethyl-2,3-dihydrobenzofuran-5-yl)sulfonyl)guanidino)phenyl)propanoate

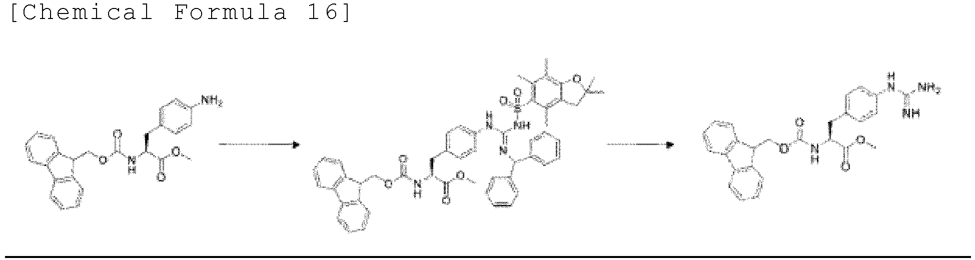

The titled compound was obtained by synthesis and purification according to known methods. The product was confirmed by 1H-NMR. The resulting compound was deprotected by the same method as in (1). The deprotection treatment was performed for 60 min, and then the production of Fmoc-F4G-Met; methyl (S)-2-((((9H-fluoren-9-yl)methoxy)carbonyl)amino)-3-(4-guanidinophenyl)propanoate was confirmed by LC/MS (Analysis condition A). As a result, the peak of Fmoc-F4G-Met had an area of 22.3%, while a peak of the titled compound in which any deprotection did not occur had an area of 12.0% in LC/MS, and a peak that represents almost the same mass (found m/z=625.4) as that of Fmoc-F4G(H,Bzh)-Met; methyl (S,E)-2-((((9H-fluoren-9-yl)methoxy)carbonyl)amino)-3-(4-(2-benzhydrylguanidino)phenyl)propanoate in which Bzh was not deprotected remained and had an area of 57.6% in LC/MS. Accordingly, it was found that the complete deprotection did not occur.

According to the above results, it was shown that the compound Fmoc-F4G(Pbf,Sub)-Met; methyl (S,Z)-2-((((9H-fluoren-9-yl)methoxy)carbonyl)amino)-3-(4-(2-(10,11-dihydro-5H-dibenzo[a,d][7]annulene-5-yl)-3-((2,2,4,6,7-pentamethyl-2,3-dihydrobenzofuran-5-yl)sulfonyl)guanidino)phenyl)propanoate had an extremely better deprotection efficiency than Fmoc-F4G(Pbf) compounds synthesized by known methods using other protecting groups.

### [Example 4]

### Synthesis of Fmoc-F3G(Pbf,Sub)-OH; (S,Z)-2-((((9H-fluoren-9-yl)methoxy)carbonyl)amino)-3-(3-(2-(10,11-dihydro-5H-dibenzo[a,d][7]annulene-5-yl)-3-((2,2,4,6,7-pentamethyl-2,3-dihydrobenzofuran-5-yl)sulfonyl)guanidino)phenyl)propanoic acid

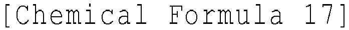

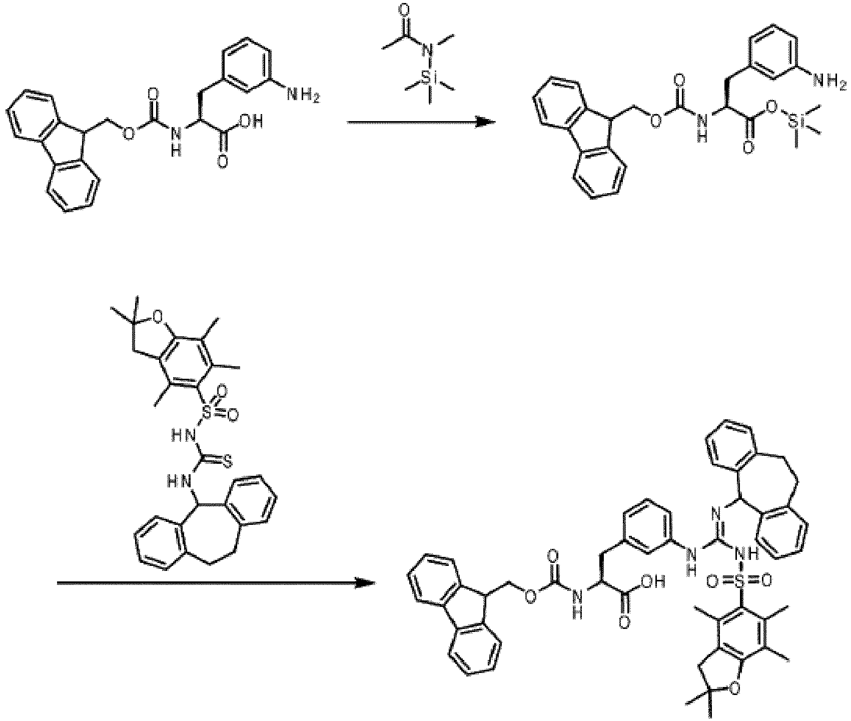

(S)-2-((((9H-fluoren-9-yl)methoxy)carbonyl)amino)-3-(3-aminophenyl)propanoic acid (1000 mg, 2.49 mmol) was suspended in THF (15 ml), and MSA (0.516 ml, 3.23 mmol) was added and dissolved by stirring for 1 hour under room temperature. To the reaction solution was added TEA (0.381 ml, 2.73 mmol), and then N-((10,11-dihydro-5H-dibenzo[a,d][7]annulene-5-yl)carbamothioyl)-2,2,4,6,7-pentamethyl-2,3-dihydrobenzofuran-5-sulfonamide (1307 mg, 2.510 mmol) and 1-(3-dimethylaminopropyl)-3-ethylcarbodiimide hydrochloride (476 mg, 2.49 mmol) were added and stirred at room temperature for 1 hour. The reaction was quenched with 0.5 N citric acid aqueous solution, and the mixture was extracted with ethyl acetate. The organic layer was washed twice with water, and then dried with sodium sulfate. The organic layer was filtered, and the filtrate was concentrated under reduced pressure. The resulting residue was dissolved in ethyl acetate, and hexane in a volume 9 times the volume of the solution was added to the solution and stirred overnight. The resulting precipitate was filtered, and the precipitate was dried to obtain the titled compound (2.1 g, 2.362 mmol, 95% yield) as pale-yellow solids. The structure was confirmed by 1H-NMR and LC/MS.
Analysis condition B: retention time=2.24 min, ESI-MS(+) found m/z=889.5 (M+H)+, calculated m/z=888.4

### [Example 5]

### Synthesis of Fmoc-Har(Pbf,Sub)-OH; (Z)-N2-(((9H-fluoren-9-yl)methoxy)carbonyl)-N6-(N'-(10,11-dihydro-5H-dibenzo[a,d] [7]annulene-5-yl) -N-((2,2,4,6,7-pentamethyl-2,3-dihydrobenzofuran-5-yl)sulfonyl)carbamimidoyl-L-lysine

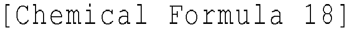

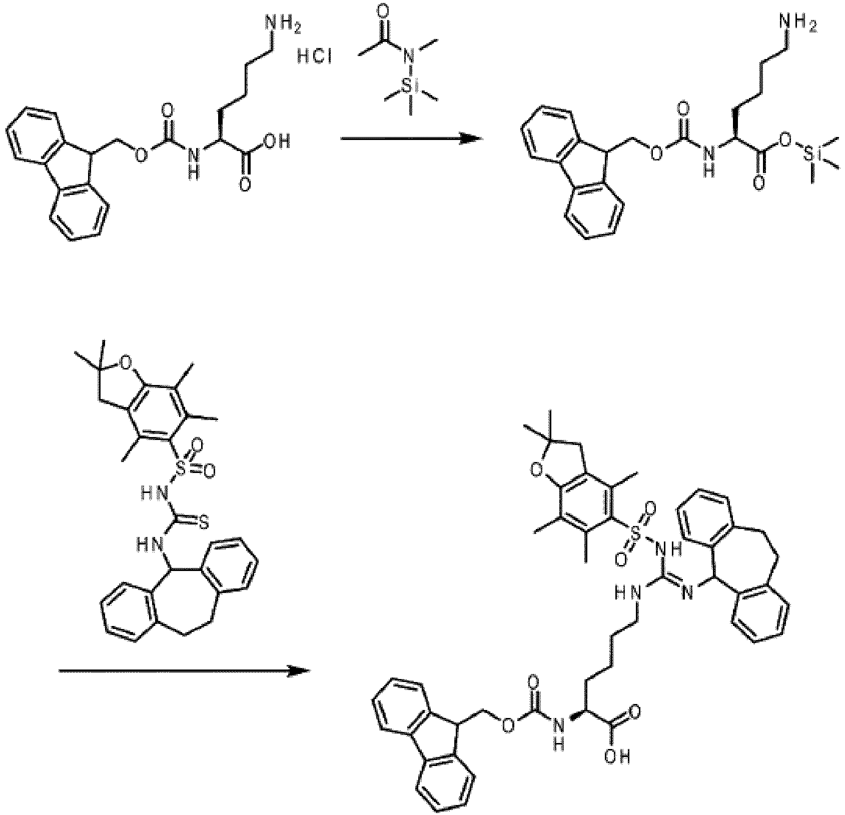

In 10 mL of THF, N-Fmoc-L-lysine hydrochloride (1.00 g, 2.47 mmol) was suspended, then MSA (0.99 mL, 6.17 mmol) was added, and the mixture was stirred at room temperature for 30 min. To the reaction solution were added N-((10,11-dihydro-5H-dibenzo [a, d] [7] annulene-5-yl) carbamothioyl) - 2,2,4,6,7-pentamethyl-2,3-dihydrobenzofuran-5-sulfonamide (1.29 g, 2.47 mmol) obtained in Example 1, and 3-(((ethylimino)methylene)amino)-N,N-dimethylpropane-1-amine hydrochloride (497 mg, 2.59 mmol), and the mixture was stirred at room temperature for 1 hour. The reaction was quenched with 10% citric acid aqueous solution, and the mixture was extracted with ethyl acetate. The resulting organic layer was washed with water and saturated sodium chloride solution, then dried with sodium sulfate, and then filtered and concentrated under reduced pressure. To the resulting residue was added ethyl acetate to dissolve the residue, and heptane was added. The resulting white solids were filtered, washed with ethyl acetate/hexane (15/85) to obtain the titled compound (1.73 g, 2.03 mmol, 82% yield). The structure was confirmed by 1H-NMR and LC/MS.
Analysis condition B: retention time=2.29 min, ESI-MS(+) found m/z=855.6 (M+H)+, calculated m/z=854.37

### [Example 6]

### Synthesis of Fmoc-GpG(Pbf,Sub)-OH; (Z)-N-(((9H-fluoren-9-yl)methoxy)carbonyl)-N-(3-(3-(10,11-dihydro-5H-dibenzo[a,d][7]annulene-5-yl)-2-((2,2,4,6,7-pentamethyl-2,3-dihydrobenzofuran-5-yl)sulfonyl)guanidino)propyl)glycine

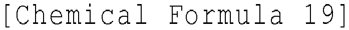

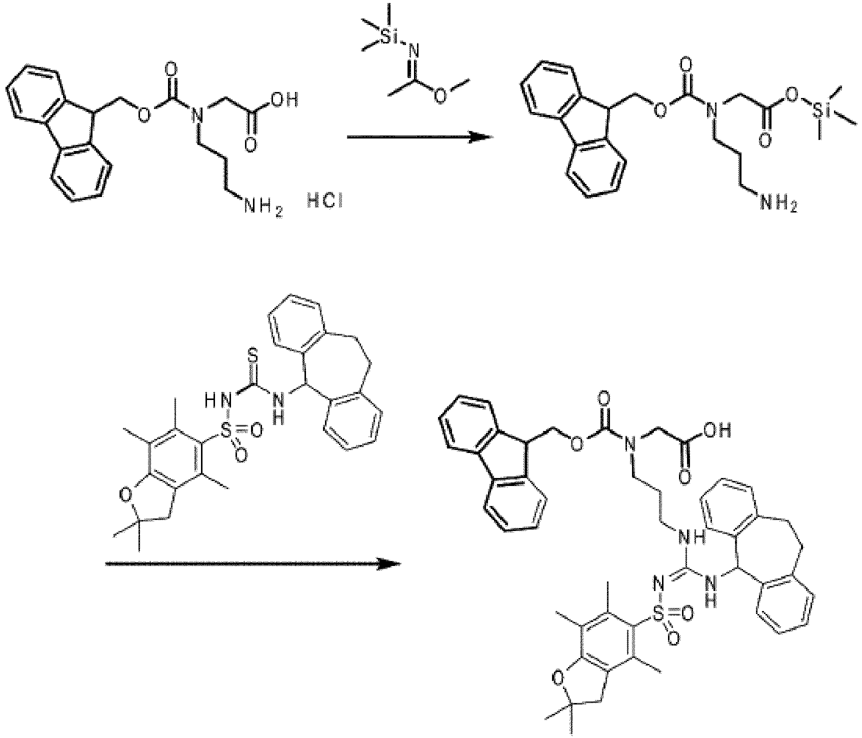

To a 100-mL round-bottom flask equipped with stirrer and a three-way cock connected to a balloon filled with nitrogen gas was added N-(((9H-fluoren-9-yl)methoxy)carbonyl)-N-(3-aminopropyl)glycine hydrochloride (3.91 g, 10.0 mmol). To the flask were added 95 mL of THF and MSA (3.82 mL, 23.81 mmol) under room temperature, and the mixture was stirred at room temperature for 30 min. To the reaction solution were added N-((10,11-dihydro-5H-dibenzo[a,d][7]annulene-5-yl)carbamothioyl)-2,2,4,6,7-pentamethyl-2,3-dihydrobenzofuran-5sulfonamide (4.96 g, 9.52 mmol) obtained in Example 1, and 3-(((ethylimino)methylene)amino)-N,N-dimethylpropane-1-amine hydrochloride (1.92 g, 10.00 mmol) under room temperature, and the mixture was stirred at room temperature for 30 min. The reaction was quenched with a saturated citric acid aqueous solution at room temperature, and the mixture was extracted with ethyl acetate. The resulting organic layer was washed with water and saturated sodium chloride solution, then dried with sodium sulfate, and then filtered and concentrated under reduced pressure. The resulting residue was suspended and washed with hexane/ethyl acetate (60 mL/30 mL), to obtain the titled compound (8.28 g, 9.84 mmol, 103% yield). The structure was confirmed by 1H-NMR and LC/MS.
Analysis condition B: retention time=2.23 min, ESI-MS(+) found m/z=841.5 (M+H)+, calculated m/z=840.36

### [Example 7]

### Synthesis of Fmoc-A4pG(Pbf,Sub)-OH; (S,Z)-2-((((9H-fluoren-9-yl)methoxy)carbonyl)amino)-3-(1-(N'-(10,11-dihydro-5H-dibenzo[a,d][7]annulene-5-yl)-N-((2,2,4,6,7-pentamethyl-2,3-dihydrobenzofuran-5-yl)sulfonyl)carbamimidoyl)piperidine-4-yl)propanoic acid

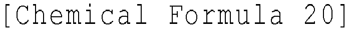

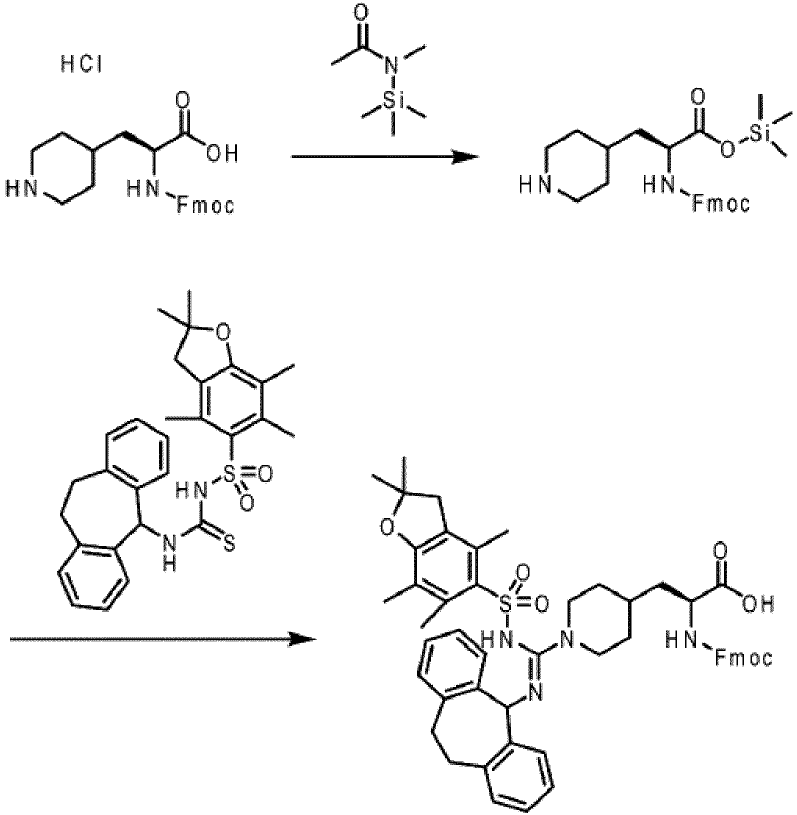

In 25 mL of THF, (S)-2-((((9H-fluoren-9-yl)methoxy)carbonyl)amino)-3-(piperidine-4-yl)propanoic acid hydrochloride (2.16 g, 5.00 mmol) was suspended, then MSA (2.81 mL, 17.50 mmol) was added, and the mixture was stirred at 0°C for 15 min. To the reaction solution were added N-((10,11-dihydro-5H-dibenzo[a,d][7]annulene-5-yl)carbamothioyl)-2,2,4,6,7-pentamethyl-2,3-dihydrobenzofuran-5-sulfonamide (2.47 g, 4.75 mmol) obtained in Example 1, and 3-(((ethylimino)methylene)amino)-N,N-dimethylpropane-1-amine hydrochloride (959 mg, 5.00 mmol), and the mixture was stirred at room temperature for 2 hours. The mixture was diluted with ethyl acetate, then the reaction was quenched with 1 N hydrochloric acid aqueous solution, and the mixture was extracted twice with ethyl acetate. The resulting organic layer was washed with water and saturated sodium chloride solution, then dried with sodium sulfate, and then filtered and concentrated under reduced pressure. The resulting residue was purified by flash column chromatography using Isolera (Biotage; Sfar HC Duo 50 g; mobile phase: hexane, 30%-100% ethyl acetate gradient) to obtain the titled compound (3.76 g, 4.00 mmol, 80% yield) as white solids. The structure was confirmed by 1H-NMR and LC/MS.
Analysis condition B: retention time=2.34 min, ESI-MS(+) found m/z=881.9 (M+H)+, calculated m/z=880.39

### [Example 8]

### Synthesis of Fmoc-F4G(Pbf,Sub)-OH; (S,Z)-2-((((9H-fluoren-9-yl)methoxy)carbonyl)amino)-3-(4-(2-(10,11-dihydro-5H-dibenzo[a,d][7]annulene-5-yl)-3-((2,2,4,6,7-pentamethyl-2,3-dihydrobenzofuran-5-yl)sulfonyl)guanidino)phenyl)propanoic acid

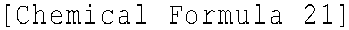

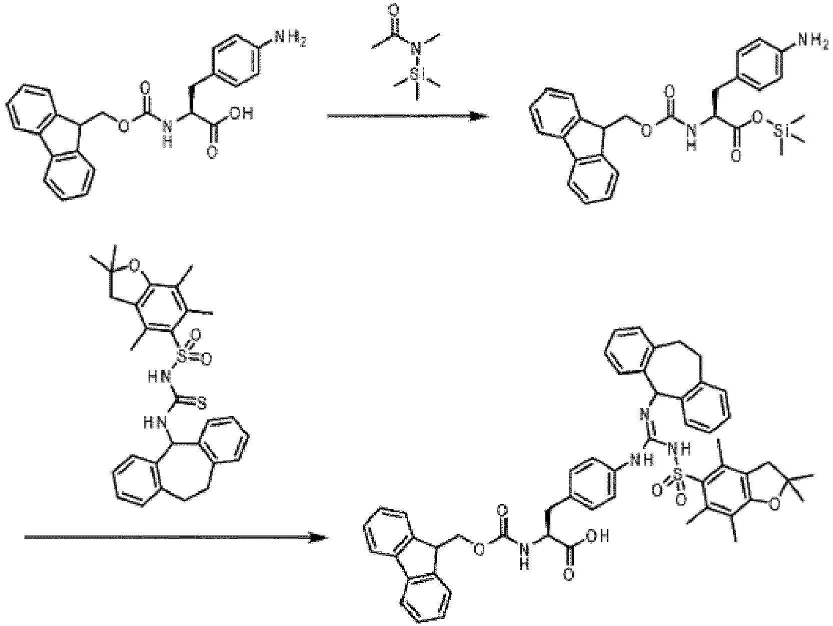

In 199 mL of THF, (S)-2-((((9H-fluoren-9-yl)methoxy)carbonyl)amino)-3-(4-aminophenyl)propanoic acid (15.0 g, 37.3 mmol) was suspended, then MSA (7.8 mL, 48.5 mmol) was added dropwise in a water bath (water bath temperature: 20.1°C to 21.4°C), and stirred for 30 min in the water bath. To the reaction solution was added N-((10,11-dihydro-5H-dibenzo[a,d][7]annulene-5-yl)carbamothioyl)-2,2,4,6,7-pentamethyl-2,3-dihydrobenzofuran-5sulfonamide (19.4 g, 37.3 mmol) obtained in Example 1 in the water bath, and thereafter a suspension prepared by adding 50 mL of dichloromethane to 1-(3-dimethylaminopropyl)-3-ethylcarbodiimide hydrochloride was added dropwise over 15 min in the water bath (water bath temperature: 20.3°C to 22.4°C). The reaction solution was stirred for 30 min in the water bath, then the reaction was quenched with 10% citric acid aqueous solution, and the mixture was extracted twice with ethyl acetate. The resulting organic layer was washed twice with water, and once with 10% sodium chloride solution, then dried with sodium sulfate, and then filtered and concentrated under reduced pressure. To the resulting residue was added 300 mL of hexane, the mixture was stirred at 50°C for 1 hour, and then gradually cooled to room temperature with stirring. The resulting pale-yellow solids were filtered, and the solids were washed with hexane to obtain the titled compound (35.1 g, 36.7 mmol, 98% yield). The structure was confirmed by 1H-NMR and LC/MS.
Analysis condition B: retention time=2.16 min, ESI-MS(+) found m/z=889.5 (M+H)+, calculated m/z=888.4

### [Example 9]

### Synthesis of Fmoc-F3G(Pbf,Sub)-OH; (S,Z)-2-((((9H-fluoren-9-yl)methoxy)carbonyl)amino)-3-(3-(2-(10,11-dihydro-5H-dibenzo[a,d] [7]annulene-5-yl) - 3-((2,2,4,6,7-pentamethyl-2,3-dihydrobenzofuran-5-yl)sulfonyl)guanidino)phenyl)propanoic acid

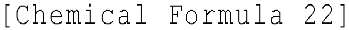

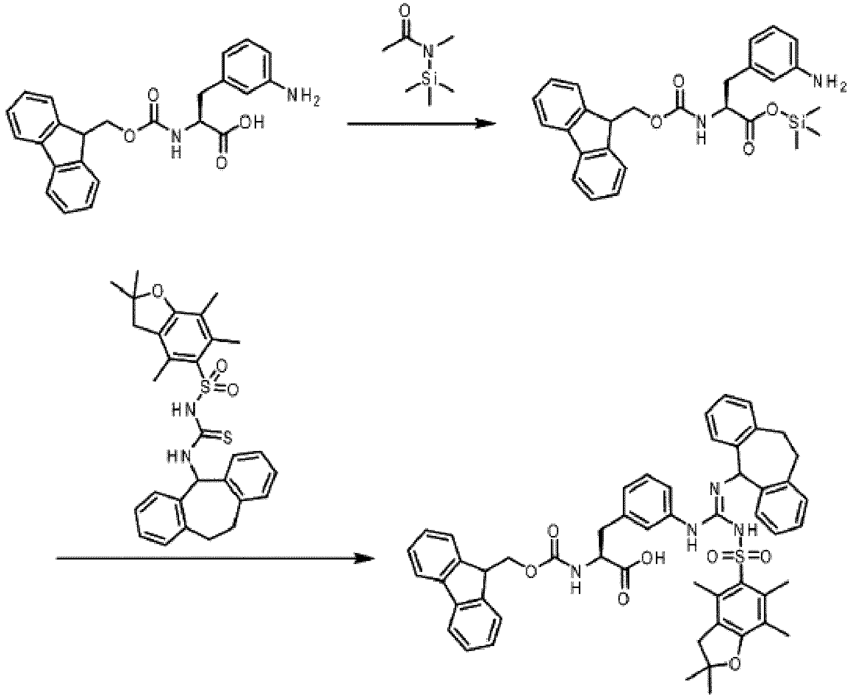

In 199 mL of THF, (S)-2-((((9H-fluoren-9-yl)methoxy)carbonyl)amino)-3-(3-aminophenyl)propanoic acid (15.0 g, 37.3 mmol) was suspended, then MSA (7.77 mL, 48.5 mmol) was added dropwise, and the mixture was stirred at room temperature for 30 min. To the reaction solution was added N-((10,11-dihydro-5H-dibenzo[a,d][7]annulene-5-yl)carbamothioyl)-2,2,4,6,7-pentamethyl-2,3-dihydrobenzofuran-5sulfonamide (19.4 g, 37.3 mmol) obtained in Example 1, and thereafter a suspension prepared by adding 49.7 mL of dichloromethane to 1-(3-dimethylaminopropyl)-3-ethylcarbodiimide hydrochloride (7.15 g, 37.3 mmol) was added dropwise over 15 min. The reaction solution was stirred at room temperature for 30 min, then the reaction was quenched with 10% citric acid aqueous solution, and the mixture was extracted with ethyl acetate. The resulting organic layer was washed twice with water, and once with saturated sodium chloride solution, then dried with sodium sulfate, and then filtered and concentrated under reduced pressure. To the resulting residue was added 200 mL of hexane, the mixture was stirred at 50°C for 1 hour, and then gradually cooled to room temperature with stirring. The resulting light brown solids were filtered, and the solids were washed with hexane to obtain the titled compound (35.0 g, 39.4 mmol, 106% yield). The structure was confirmed by 1H-NMR and LC/MS.
Analysis condition B: retention time=2.18 min, ESI-MS(+) found m/z=889.5 (M+H)+, calculated m/z=888.4

### [Example 10]

The following compounds were synthesized as in Examples 5 to 9. The compounds synthesized are shown in Table 1. The structures of the compounds synthesized were confirmed by 1H-NMR and LC/MS (Analysis condition B).

**[Table 1]**

| | Name | Exact Name | Structure | m/z (M+H)+ |
|---|---|---|---|---|
| 1 | Fmoc-HarMe(Pbf,Sub)-OH | (Z)-N2-(((9H-fluoren-9-yl)methoxy)carbonyl)-N6-(N'-(10,11-dihydro-5H-dibenzo[a,d][7]annulene-5-yl)-N-((2,2,4,6,7-pentamethyl-2,3-dihydrobenzofuran-5-yl)sulfonyl)carbamimidoyl)-N6-methyl-L-lysine | 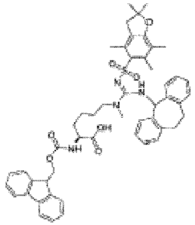 | 869.5 |
| 2 | Fmoc-RMe(Pbf,Sub)-OH | (Z)-N2-(((9H-fluoren-9-yl)methoxy)carbonyl)-Nw'-(10,11-dihydro-5H-dibenzo[a,d][7]annulene-5-yl)-Nd-methyl-Nw-((2,2,4,6,7-pentamethyl-2,3-dihydrobenzofuran-5-yl)sulfonyl)-L-arginine | 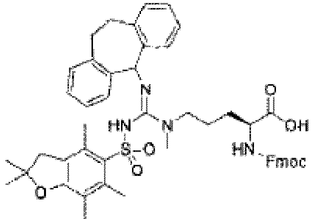 | 855.6 |
| 3 | Fmoc-A4pipG (Pbf, Sub)-OH | (Z)-4-((((9H-fluoren-9-yl)methoxy)carbonyl)amino)-1-(N'-(10,11-dihydro-5H-dibenzo[a,d][7]annulene-5-yl)-N-((2,2,4,6,7-pentamethyl-2,3-dihydrobenzofuran-5-yl)sulfonyl)carbamimidoyl)piperidine-4-carboxylic acid | 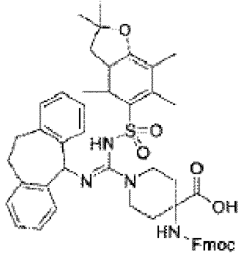 | 853.6 |
| 4 | Fmoc-G4pipG(Pbf,Sub)-OH | (S,Z)-2-((((9H-fluoren-9-yl)methoxy)carbonyl)amino)-2-(1-(N'-(10,11-dihydro-5H-dibenzo[a,d][7]annulene-5-yl)-N-((2,2,4,6,7-pentamethyl-2,3-dihydrobenzofuran-5-yl)sulfonyl)carbamimidoyl)piperidine-4-yl)acetic acid | 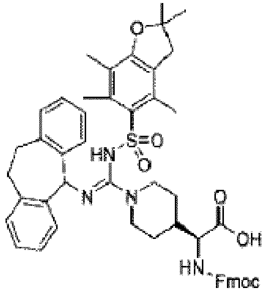 | 867.5 |
| 5 | Fmoc-Nar(Pbf,Sub)-OH | (S,Z)-2-((((9H-fluoren-9-yl)methoxy)carbonyl)amino)-4-(2-(10,11-dihydro-5H-dibenzo[a,d][7]annulene-5-yl)-3-((2,2,4,6,7-pentamethyl-2,3-dihydrobenzofuran-5-yl)sulfonyl)guanidino)butanoic acid | 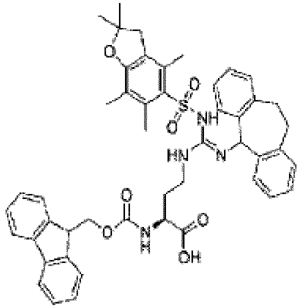 | 828 |
| 6 | Fmoc-dharMe(Pbf,Sub)-OH | (Z)-N2-(((9H-fluoren-9-yl)methoxy)carbonyl)-N6-(N-(10,11-dihydro-5H-dibenzo[a,d][7]annulene-5-yl)-N'-((2,2,4,6,7-pentamethyl-2,3-dihydrobenzofuran-5-yl)sulfonyl)carbamimidoyl)-N6-methyl-D-lysine | 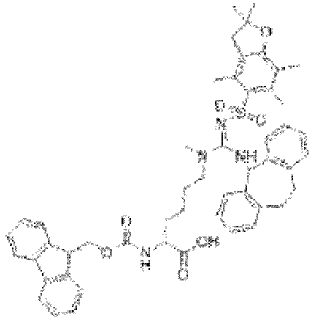 | 869.3 |
| 7 | Fmoc-df4G(Pbf,Sub)-OH | (R,E)-2-((((9H-fluoren-9-yl)methoxy)carbonyl)amino)-3-(4-(3-(10,11-dihydro-5H-dibenzo[a,d][7]annulene-5-yl)-2-((2,2,4,6,7-pentamethyl-2,3-dihydrobenzofuran-5-yl)sulfonyl)guanidino)phenyl)propanoic acid | 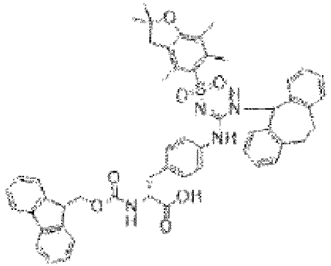 | 889.3 |
| 8 | Fmoc-df3G(Pbf,Sub)-OH | (R,Z)-2-((((9H-fluoren-9-yl)methoxy)carbonyl)amino)-3-(3-(3-(10,11-dihydro-5H-dibenzo[a,d][7]annulene-5-yl)-2-((2,2,4,6,7-pentamethyl-2,3-dihydrobenzofuran-5-yl)sulfonyl)guanidino)phenyl)propanoic acid | 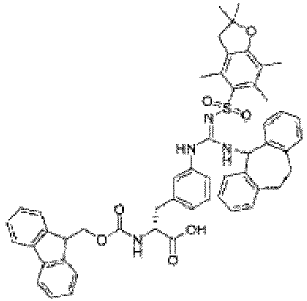 | 889.3 |
| 9 | Fmoc-drMe(Pbf,Sub)-OH | (Z)-N2-(((9H-fluoren-9-yl)methoxy)carbonyl)-Nw-(10,11-dihydro-5H-dibenzo[a,d][7]annulene-5-yl)-Nd-methyl-Nw'-((2,2,4,6,7-pentamethyl-2,3-dihydrobenzofuran-5-yl)sulfonyl)-D-arginine | 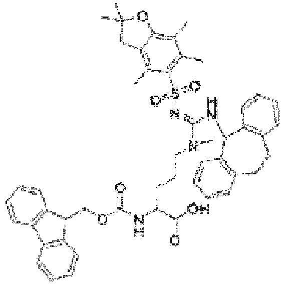 | 855.5 |

### INDUSTRIAL APPLICABILITY

The present invention relates to a guanidine derivative and others, and thus can be utilized in the fields of chemistry, biochemistry, pharmaceutical and polymer chemistry.

## Claims

1. A compound represented by the following Formula (I), a tautomer thereof, an enantiomer thereof, a pharmaceutically acceptable salt thereof, or a pharmaceutically acceptable solvate thereof: 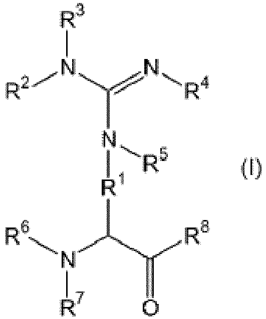 in Formula (I),
-R¹- represents a C₁-C₁₀ alkylene group which may have one or more substituent groups selected from the group A, a C₆₋₁₀ arylene group which may have one or more substituent groups selected from the group A, or a group represented by -R¹¹-R¹²-,
the group A consists of a halogen atom, an amino group, a nitro group, a C₁₋₅ alkyl group, a C₁₋₃ alkoxy group, a C₁₋₃ alkylthio group, and a C₁₋₃ halogenoalkyl group,
-R¹¹- represents a C₆₋₁₀ arylene group which may have one or more substituent groups selected from the group A,
the group represented by -R¹²- represents a C₁-C₄ alkylene group which may have one or more substituent groups selected from the group A,
R² and R⁴ may be the same or different and represent a protecting group of an amino acid or a hydrogen atom,
R³ represents a hydrogen atom,
R⁵ represents a hydrogen atom, a halogen atom, a hydroxy group, an amino group, a nitro group, a C₁-₅ alkyl group, a C₁₋₃ alkoxy group, a C₁₋₃ alkylthio group, or a C₁₋₃ halogenoalkyl group, or R⁵ is taken together with -N-R¹- to form a C₅₋₇ heterocyclic amine which may have an asymmetric carbon atom at the alpha-position,
R⁶ and R⁷ may be the same or different and represent a protecting group of an amino acid, a C₁-₅ alkyl group or a hydrogen atom, and
R⁸ represents a protecting group of an amino acid, a hydrogen atom, a halogen atom, a hydroxy group, an amino group, a nitro group, a C₁-₅ alkyl group, a C₁₋₃ alkoxy group, a C₁₋₃ alkylthio group, a C₁₋₃ halogenoalkyl group or a group represented by -O-R¹³-, and
the group represented by R¹³- represents a protecting group of an oxygen atom, a C₁-₅ alkyl group, a C₁₋₃ alkoxy group, a C₁₋₃ alkylthio group, or a C₁₋₃ halogenoalkyl group,
with the proviso that a compound in which -R¹- represents a propylene group, R² to R⁷ each represent a hydrogen atom, and R⁸ represents a hydroxy group (arginine), and
a compound in which -R¹- represents a group represented by -R¹¹-R¹²-, -R¹¹- represents a 1,4-phenylene group, -R¹²- represents a methylene group, R², R⁴, R⁵, R⁶, and R⁷ each represents a hydrogen atom, and R⁸ represents a hydroxy group are excluded.

2. The compound according to claim 1, a tautomer thereof, an enantiomer thereof, a pharmaceutically acceptable salt thereof, or a pharmaceutically acceptable solvate thereof, wherein
R⁵ represents a hydrogen atom, a halogen atom, a hydroxy group, an amino group, a nitro group, a C₁-₅ alkyl group, a C₁₋₃ alkoxy group, a C₁₋₃ alkylthio group, or a C₁₋₃ halogenoalkyl group, and
R⁸ represents a hydrogen atom, a halogen atom, a hydroxy group, an amino group, a nitro group, a C₁-₅ alkyl group, a C₁₋₃ alkoxy group, a C₁₋₃ alkylthio group, a C₁₋₃ halogenoalkyl group or a group represented by - O-R¹³- .

3. The compound according to claim 2, a tautomer thereof, an enantiomer thereof, a pharmaceutically acceptable salt thereof, or a pharmaceutically acceptable solvate thereof, wherein
-R¹- represents a group represented by -R¹¹-R¹²-,
-R¹¹- represents a 1,3-phenylene group or a 1,4-phenylene group which may have one or more substituent groups selected from the group A, and
the group represented by -R¹²- represents a C₁₋₄ alkylene group.

4. The compound according to claim 3, a tautomer thereof, an enantiomer thereof, a pharmaceutically acceptable salt thereof, or a pharmaceutically acceptable solvate thereof, wherein
R⁵ represents a hydrogen atom, a methyl group, or an ethyl group,
R⁶ represents an Fmoc group (9-fluorenylmethyloxycarbonyl group),
R⁷ represents a hydrogen atom, and
R⁸ represents a hydroxy group, or a group represented by -O-R¹³-, and
the group represented by R¹³- represents a protecting group of an oxygen atom, a methyl group, or an ethyl group.

5. The compound according to claim 2, a tautomer thereof, an enantiomer thereof, a pharmaceutically acceptable salt thereof, or a pharmaceutically acceptable solvate thereof, wherein
-R¹- represents a C₃-C₆ alkylene group.

6. The compound according to claim 5, a tautomer thereof, an enantiomer thereof, a pharmaceutically acceptable salt thereof, or a pharmaceutically acceptable solvate thereof, wherein
R⁵ represents a hydrogen atom, a methyl group, or an ethyl group,
R⁶ represents an Fmoc group (9-fluorenylmethyloxycarbonyl group),
R⁷ represents a hydrogen atom, a methyl group, or an ethyl group, and
R⁸ represents a hydroxy group, or a group represented by -O-R¹³-, and
the group represented by R¹³- represents a protecting group of an oxygen atom, a methyl group, or an ethyl group.

7. The compound according to claim 2, a tautomer thereof, an enantiomer thereof, a pharmaceutically acceptable salt thereof, or a pharmaceutically acceptable solvate thereof, wherein
R² and R⁴ represents any one selected from the group consisting of Pbf (2,2,4,6,7-pentamethyl-2,3-dihydrobenzofuran-5-sulfonyl group), Pmc (2,2,5,7,8-pentamethylchroman-6-sulfonyl group) and Sub (10,11-dihydro-5H-dibenzo-[a,d] [7]annulene-5-yl group) and a hydrogen atom.

8. A method for manufacture of a peptide using a compound according to claim 1, a tautomer thereof, an enantiomer thereof, a pharmaceutically acceptable salt thereof, or a pharmaceutically acceptable solvate thereof.

9. A method for manufacture of a compound represented by the following Formula (I), a tautomer thereof, an enantiomer thereof, a pharmaceutically acceptable salt thereof, or a pharmaceutically acceptable solvate thereof, the method comprising a mixing step in which a compound represented by the following Formula (II) is mixed with a compound represented by the following Formula (III): 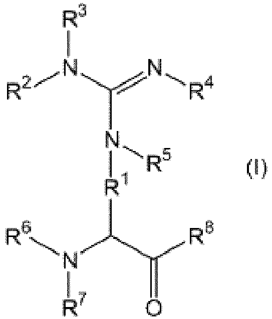 in Formula (1),
-R¹- represents a C₁-C₁₀ alkylene group which may have one or more substituent groups selected from the group A, a C₆₋₁₀ arylene group which may have one or more substituent groups selected from the group A, or a group represented by -R¹¹-R¹²-,
the group A consists of a halogen atom, an amino group, a nitro group, a C₁₋₅ alkyl group, a C₁₋₃ alkoxy group, a C₁₋₃ alkylthio group, and a C₁₋₃ halogenoalkyl group,
-R¹¹- represents a C₆₋₁₀ arylene group which may have one or more substituent groups selected from the group A,
the group represented by -R¹²- represents a C₁-C₄ alkylene group which may have one or more substituent groups selected from the group A,
R² and R⁴ may be the same or different and represent a protecting group of an amino acid or a hydrogen atom,
R³ represents a hydrogen atom,
R⁵ represents a hydrogen atom, a halogen atom, a hydroxy group, an amino group, a nitro group, a C₁-₅ alkyl group, a C₁₋₃ alkoxy group, a C₁₋₃ alkylthio group, or a C₁₋₃ halogenoalkyl group, or R⁵ is taken together with -N-R¹- to form a C₅₋₇ heterocyclic amine which may have an asymmetric carbon atom,
R⁶ and R⁷ may be the same or different and represent a protecting group of an amino acid, a C₁-₅ alkyl group or a hydrogen atom,
R⁸ represents a protecting group of an amino acid, a hydrogen atom, a halogen atom, a hydroxy group, an amino group, a nitro group, a C₁-₅ alkyl group, a C₁₋₃ alkoxy group, a C₁₋₃ alkylthio group, a C₁₋₃ halogenoalkyl group or a group represented by -O-R¹³-, and
the group represented by R¹³- represents a protecting group of an oxygen atom, a C₁-₅ alkyl group, a C₁₋₃ alkoxy group, a C₁₋₃ alkylthio group, or a C₁₋₃ halogenoalkyl group,
with the proviso that a compound in which -R¹- represents a propylene group, R² to R⁷ each represent a hydrogen atom, and R⁸ represents a hydroxy group, and
a compound in which -R¹- represents a group represented by -R¹¹-R¹²-, -R¹¹- represents a 1,4-phenylene group, -R¹²- represents a methylene group, R², R⁴, R⁵, R⁶, and R⁷ each represents a hydrogen atom, and R⁸ represents a hydroxy group are excluded, 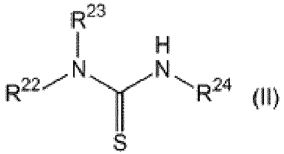 in formula (II), R²², R²³ and R²⁴ have the same meaning as R², R³ and R⁴, respectively, 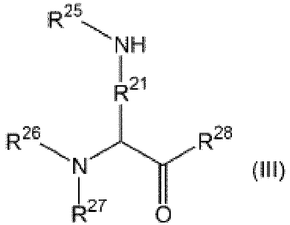 in Formula (III),
R²¹, R²⁵, R²⁶, and R²⁷ have the same meaning as R¹, R⁵, R⁶, and R⁷, respectively,
R²⁸ represents a group represented by -O-R³³-, and
the group represented by R³³- represents a protecting group of an oxygen atom.

10. The method according to claim 9, wherein
-R¹- represents a group represented by -R¹¹-R¹²-,
-R¹¹- represents a 1,3-phenylene group or a 1,4-phenylene group which may have one or more substituent groups selected from the group A,
the group represented by -R¹²- represents a C₁₋₄ alkylene group,
R⁵ represents a hydrogen atom, a methyl group, or an ethyl group,
R⁶ represents an Fmoc group (9-fluorenylmethyloxycarbonyl group),
R⁷ represents a hydrogen atom, and
R⁸ represents a hydroxy group, or a group represented by -O-R¹³-, and
the group represented by R¹³- represents a protecting group of an oxygen atom, a methyl group, or an ethyl group.

11. The method according to claim 9, wherein
R²² and R²⁴ represents any one selected from the group consisting of Pbf (2,2,4,6,7-pentamethyl-2,3-dihydrobenzofuran-5-sulfonyl group), Pmc (2,2,5,7,8-pentamethylchroman-6-sulfonyl group) and Sub (10,11-dihydro-5H-dibenzo- [a,d] [7]annulene-5-yl group) and a hydrogen atom.

12. A method for manufacture of a peptide, the method comprising
a compound manufacturing step in which a compound represented by Formula (I), a tautomer thereof, an enantiomer thereof, a pharmaceutically acceptable salt thereof, or a pharmaceutically acceptable solvate thereof is manufactured according to the method according to claim 9, and
a peptide manufacture step in which a peptide is manufactured using the compound represented by Formula (I), a tautomer thereof, an enantiomer thereof, a pharmaceutically acceptable salt thereof, or a pharmaceutically acceptable solvate thereof manufactured in the compound manufacture step.

13. A compound represented by formula (IV) or (V):

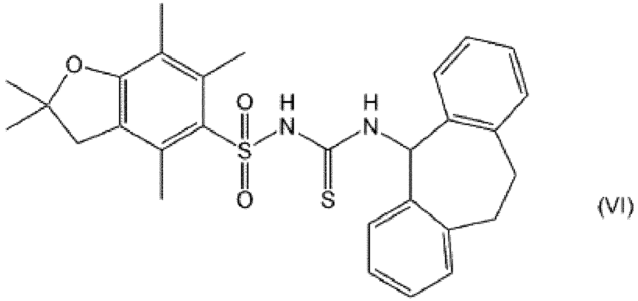

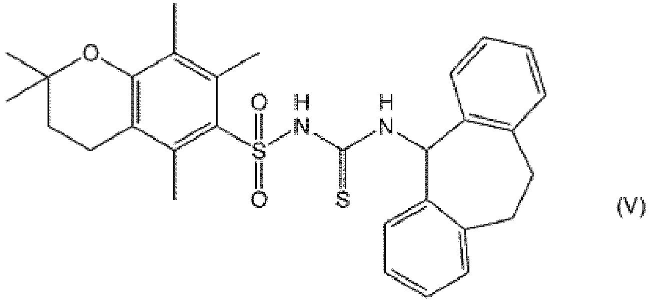

14. An amino acid selected from the group consisting of the following, or a salt thereof or a solvate thereof:
i. 2-((((9H-fluoren-9-yl)methoxy)carbonyl)amino)-3-(4-(2-(10,11-dihydro-5H-dibenzo[a,d][7]annulene-5-yl)-3-((2,2,4,6,7-pentamethyl-2,3-dihydrobenzofuran-5-yl)sulfonyl)guanidino)phenyl)propanoic acid;
ii. 2-((((9H-fluoren-9-yl)methoxy)carbonyl)amino)-3-(3-(2-(10,11-dihydro-5H-dibenzo[a,d][7]annulene-5-yl)-3-((2,2,4,6,7-pentamethyl-2,3-dihydrobenzofuran-5-yl)sulfonyl)guanidino)phenyl)propanoic acid;
iii. N2-(((9H-fluoren-9-yl)methoxy)carbonyl)-N6-(N'-(10,11-dihydro-5H-dibenzo[a,d][7]annulene-5-yl)-N-((2,2,4,6,7-pentamethyl-2,3-dihydrobenzofuran-5-yl)sulfonyl)carbamimidoyl-L-lysine;
iv. N-(((9H-fluoren-9-yl)methoxy)carbonyl)-N-(3-(3-(10,11-dihydro-5H-dibenzo[a,d][7]annulene-5-yl)-2-((2,2,4,6,7-pentamethyl-2,3-dihydrobenzofuran-5-yl) sulfonyl)guanidino)propyl)glycine;
v. 2-((((9H-fluoren-9-yl)methoxy)carbonyl)amino)-3-(1-(N'-(10,11-dihydro-5H-dibenzo[a,d][7]annulene-5-yl)-N-((2,2,4,6,7-pentamethyl-2,3-dihydrobenzofuran-5-yl)sulfonyl)carbamimidoyl)piperidine-4-yl)propanoic acid;
vi. N2-(((9H-fluoren-9-yl)methoxy)carbonyl)-N6-(N'-(10,11-dihydro-5H-dibenzo[a,d][7]annulene-5-yl)-N-((2,2,4,6,7-pentamethyl-2,3-dihydrobenzofuran-5-yl)sulfonyl)carbamimidoyl)-N6-methyl-L-lysine;
vii. N2-(((9H-fluoren-9-yl)methoxy)carbonyl)-Nw'-(10,11-dihydro-5H-dibenzo[a,d][7]annulene-5-yl)-Nd-methyl-Nw-((2,2,4,6,7-pentamethyl-2,3-dihydrobenzofuran-5-yl)sulfonyl)-L-arginine;
viii. 4-((((9H-fluoren-9-yl)methoxy)carbonyl)amino)-1-(N'-(10,11-dihydro-5H-dibenzo[a,d][7]annulene-5-yl)-N-((2,2,4,6,7-pentamethyl-2,3-dihydrobenzofuran-5-yl)sulfonyl)carbamimidoyl)piperidine-4-carboxylic acid;
ix. 2-((((9H-fluoren-9-yl)methoxy)carbonyl)amino)-2-(1-(N'-(10,11-dihydro-5H-dibenzo[a,d][7]annulene-5-yl)-N-((2,2,4,6,7-pentamethyl-2,3-dihydrobenzofuran-5-yl)sulfonyl)carbamimidoyl)piperidine-4-yl)acetic acid;
x. 2-((((9H-fluoren-9-yl)methoxy)carbonyl)amino)-4-(2-(10,11-dihydro-5H-dibenzo[a,d][7]annulene-5-yl)-3-((2,2,4,6,7-pentamethyl-2,3-dihydrobenzofuran-5-yl)sulfonyl)guanidino)butanoic acid;
xi. N2-(((9H-fluoren-9-yl)methoxy)carbonyl)-N6-(N- (10, 11-dihydro-5H-dibenzo [a,d] [7] annulene-5-yl)-N'-((2,2,4,6,7-pentamethyl-2,3-dihydrobenzofuran-5-yl)sulfonyl)carbamimidoyl)-N6-methyl-D-lysine;
xii. 2-((((9H-fluoren-9-yl)methoxy)carbonyl)amino)-3-(4-(3-(10,11-dihydro-5H-dibenzo[a,d][7]annulene-5-yl)-2-((2,2,4,6,7-pentamethyl-2,3-dihydrobenzofuran-5-yl)sulfonyl)guanidinophenyl)propanoic acid;
xiii. 2-((((9H-fluoren-9-yl)methoxy)carbonyl)amino)-3-(3-(3-(10,11-dihydro-5H-dibenzo[a,d][7]annulene-5-yl)-2-((2,2,4,6,7-pentamethyl-2,3-dihydrobenzofuran-5-yl)sulfonyl)guanidino)phenyl)propanoic acid; and
xiv. N2-(((9H-fluoren-9-yl)methoxy)carbonyl)-Nw-(10,11-dihydro-5H-dibenzo[a,d][7]annulene-5-yl)-Nd-methyl-Nw'-((2,2,4,6,7-pentamethyl-2,3-dihydrobenzofuran-5-yl)sulfonyl)-D-arginine.
